# EUROPEAN PATENT APPLICATION

(11) **EP 4 206 212 A1**
(43) Date of publication of application: **05.07.2023**
(21) Application number: 20955749.5
(22) Date of filing: 30.09.2020
(51) Int. Cl.: C07H 7/04, C07D 409/10, C07D 309/10, A61K 31/7042, A61P 3/10, A61P 13/12, A61P 9/00

(54) **SGLT-2 INHIBITOR SARCOSINE CO-CRYSTAL, PREPARATION METHOD THEREFOR AND USE THEREOF**

(71) Applicant: Beijing Creatron Institute of Pharmaceutical Research Co., Ltd., Beijing 102200 (CN)
(72) Inventor: JIA, Huijuan, Beijing 102200 (CN); HOU, Wei, Beijing 102200 (CN); WANG, Yanxin, Tianjin 300457 (CN); LI, Yan, Tianjin 300457 (CN); REN, Xiaohui, Beijing 102200 (CN); JIA, Tiange, Tianjin 300457 (CN)
(74) Representative: Prüfer & Partner mbB Patentanwälte · Rechtsanwälte
(86) International application number: PCT/CN2020/119547
(87) International publication number: WO 2022/067724

(57) **Abstract**

Provided are an SGLT-2 inhibitor-sarcosine cocrystal, a preparation method therefor and use thereof. Using sarcosine as a ligand, said cocrystal has higher safety and lower costs; a drug cocrystal has higher stability, and during the production process or storage process of a formulation composition, the crystal form is not easily changed; said cocrystal does not melt when heated, does not stick, aggregate or generate static electricity, and has better mixing uniformity. Said cocrystal has uniform distribution in a prepared pharmaceutical composition, causing that the pharmaceutical composition has better in-vivo release, absorption and efficacy, and has small difference between batches; and the pharmaceutical composition has high stability, and is more conducive to storage and transportation. The present invention has a simple preparation process, has high reproducibility, has short crystallization time, has low process condition requirements, and has higher economic benefits; the present invention does not use an unsafe solvent during the preparation process; and a crude product or intermediate of the SGLT-2 inhibitor can be simultaneously purified.

## Description

### FIELD

The present invention relates to the technical field of chemical pharmacy, in particular to an SGLT-2 inhibitor-sarcosine cocrystal, a preparation method therefor and an application thereof.

### BACKGROUND

According to the IDF Diabetes Atlas 9^{th} edition released by the International Diabetes Federation, 463 million people aged 20-79 are living with diabetes worldwide, most of which are type 2 diabetes. This number is predicted to rise to 578 million (10.2%) by 2030 and 700 million (10.9%) by 2045. The IDF reports that 32% of diabetic patients worldwide suffer from cardiovascular disease; more than 80% of end-stage renal disease is caused by diabetes or hypertension or both; and diabetic foot and lower limb complications affect 40 million to 60 million diabetic patients.

Sodium-dependent glucose transporter 2 (SGLT-2) inhibitors can inhibit the reabsorption of glucose by the kidneys, and excrete excess glucose from the urine, so as to achieve the purpose of lowering blood sugar. The currently marketed SGLT-2 inhibitors (by trade name) include Faxiga (with dapagliflozin as the active ingredient), Invokana (with canagliflozin as the active ingredient), Jardiance (with empagliflozin as the active ingredient), Suglat (with ipagliflozin as the active ingredient) and Tofogliflozin preparations. Among the above-mentioned SGLT-2 inhibitors, Faxiga, Invokana and Jardiance have been approved by the US FDA and EMA for many years. Their clinical efficacy and safety, combining with diet and exercise, have been demonstrated to improve blood sugar control in adults with type 2 diabetes and to reduce risk of hospitalization due to heart failure in adults with type 2 diabetes and cardiovascular disease or multiple cardiovascular risk factors.

In prior art, WO03099836A1/CN100534997C discloses a method for synthesizing and purifying dapagliflozin, which obtains dapagliflozin as an amorphous glassy solid. This glassy solid tends to soften and form an oily substance when heated, and tends to absorb moisture and deteriorate. As a result, the active ingredient fails to be evenly distributed in a product to be used in the production of preparations. WO2008/002824A1/US7919598B2/CN1014792878 discloses the preparation and physicochemical properties of a pharmaceutically acceptable crystal form of dapagliflozin (S)-propylene glycol hydrate crystal form used in Faxiga, accompanied by desolvation at 45°C-100°C. US8513202/CN101573368B discloses a pharmaceutically acceptable crystal form of canagliflozin hemihydrate used in Invokana, with a melting point of 97°C-100°C; WO2014159151A1 discloses the preparation of a pharmaceutically acceptable crystal form of topagliflozin, and the crystal form is a hydrate with a melting point of 71°C-92°C. In order to adapt to the production of preparations, the crystal form of the above-mentioned active ingredients cannot be subjected to the powder direct compression process in the production process of the preparation composition, otherwise there will be problems such as uneven mixing, sticking, and content not meeting the requirements due to electrostatic adsorption.

Pharmaceutical cocrystal, a crystal formed by the combination of drug molecules and cocrystal reagents under the action of hydrogen bonds or other non-covalent bonds, is currently a focus and frontier of research of the international crystal engineering.

Studies have shown that dapagliflozin-L-proline cocrystal has polymorphs, and its preparation process conditions are relatively harsh: three days of crystallization at -20°C. It requires high energy consumption for low temperature control, and in addition, the step of cocrystal formation alone takes three days, which delays the entire production cycle. Topagliflozin-L-proline cocrystal is not as good as its hydrate crystal form with low melting point in terms of crystal form stability, hygroscopicity, static electricity, fluidity, and chemical stability. Therefore, current marketed product does not use topagliflozin-L-proline cocrystal, but its hydrate crystal form with a melting point of 71°C-92°C.

In addition, ligands or solvents used in existing pharmaceutically acceptable crystal form or cocrystal of SGLT-2 inhibitors are chiral reagents or chiral ligands, which are expensive, resulting in a relatively high cost for the final obtained drug.

The SGLT-2 inhibitors disclosed in the prior art require some special purification means before the process steps of final crystallization, salt formation, and cocrystal preparation to purify the crude product or intermediate of the SGLT-2 inhibitor to a purity of more than 99%, followed by subsequent crystallization, salt formation, and cocrystal formation steps. In particular, the difficulty in removing the process impurities, such as ring-opening impurities, five-membered ring impurities, diastereoisomers, dimers, and a large number of non-specific impurities, produced in the preparation process of SGLT-2 inhibitors by simple solvent recrystallization necessitates the introduction of a separate purification step in the process, which not only prolongs the production cycle, but also significantly increases the production cost, and it is hard to ensure the reproducibility and stability of the quality between batches in the mass production process.

In summary, for the SGLT-2 inhibitor, the development of a pharmaceutically acceptable crystal form that is more conducive to the production of preparations, simple preparation of crystal forms, available reproducibility, low cost, stable quality and safety is of great significance to reduce the cost of clinical medication and improve the safety, efficacy and controllability of marketed products.

### SUMMARY

In view of this, the technical problem to be solved by the present invention is to provide an SGLT-2 inhibitor sarcosine cocrystal, a preparation method therefor and an application thereof, to prepare a cocrystal of SGLT-2 inhibitor which has a simple process, high reproducibility, controllable crystal form and quality, suitable for large-scale production of preparation compositions, good stability in the storage of raw materials and production process, and strong impurity removal ability.

To achieve the above purposes, the present invention provides an SGLT-2 inhibitor-sarcosine cocrystal.

In the present invention, the SGLT-2 inhibitor comprises a classical glucoside structure and can form a cocrystal with sarcosine. Preferably, the SGLT-2 inhibitor include but is not limited to dapagliflozin, empagliflozin, canagliflozin, tofogliflozin, ipragliflozin, luseogliflozin, sotagliflozin, janagliflozin, bexagliflozin, alligliflozin, ertugliflozin, henagliflozin, remogliflozin, tianagliflozin, wangeliejing, and

(2'R,3'R,4'S,5'S,6'R)-6-(4-ethoxybenzyl)-6'-(hydroxymethyl)-7-methyl-3',4',5',6'-tetrahydrospiro[ benzo[d][1,3]dioxine-4,2'-pyran]-3',4',5'-triol, and the specific structural formula are as shown in Table 1:

**Table 1 Structure and naming of SGLT-2 inhibitors**

| **No.** | **Chemical structural formula** | **Common name** | **Chemical name** |
|---|---|---|---|
| 1 | 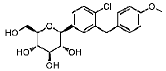 | Dapagliflozi n | (2S,3R,4R,5S,6R)-2-[ 4-chloro-3- [(4-ethoxy phenyl) methyl]phenyl]-6-(hy droxymethyl)oxane-3, 4,5-triol |
| 2 | 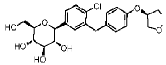 | Empaglifloz in | (2S,3R,4R,5S,6R)-2-[ 4-chloro-3- [[4-[(3 S)-o xolan-3-yl]oxyphenyl ]methyl]phenyl]-6-(hy droxymethyl)oxane-3, 4,5-triol |
| 3 | 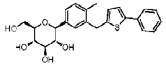 | Canagliflozi n | (2S,3R,4R,5S,6R)-2-[ 3-[[5-(4-fluorophenyl) thiophen-2-yl]methyl] -4-methylphenyl]-6-(h ydroxymethyl)oxane-3,4,5-triol |
| 4 | 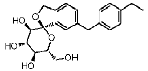 | Tofogliflozi n | (3S,3'R,4'S,5'S,6'R)-5 -[(4-ethylphenyl)meth yl]-6'-(hydroxymethyl )spiro[1H-2-benzofur an-3,2'-oxane]-3',4',5'-triol |
| 5 | 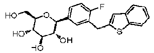 | Ipragliflozin | (2S,3R,4R,5S,6R)-2-[ 3-(1-benzothiophen-2 -ylmethyl)-4-fluoroph enyl]-6-(hydroxymeth yl)oxane-3,4,5-triol |
| 6 | 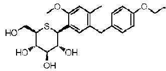 | Luseogliflo zin | (2S,3R,4R,5S,6R)-2-[ 5-[(4-ethoxyphenyl)m ethyl]-2-methoxy-4-m ethylphenyl]-6-(hydro xymethyl)thiane-3,4,5 -triol |
| 7 | 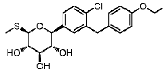 | Sotagliflozi n | (2S,3R,4R,5S,6R)-2-[ 4-chloro-3-[(4-ethoxy phenyl)methyl]phenyl ]-6-methylsulfanyloxa ne-3,4,5-triol |
| 8 | 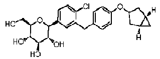 | Janagliflozi n | (2S,3R,4R,5S,6R)-2-[ 3-[[4-[[(1 R,5S)-3-bicy clo[3.1.0]hexanyl]oxy ]phenyl]methyl]-4-chl orophenyl]-6-(hydrox ymethyl)oxane-3,4,5-t riol |
| 9 | 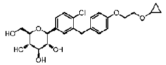 | Bexagliflozi n | (2S,3R,4R,5S,6R)-2-[ 4-chloro-3-[[4-(2-cycl opropyloxyethoxy)ph enyl]methyl]phenyl]-6-(hydroxymethyl)ox ane-3,4,5-triol |
| 10 | 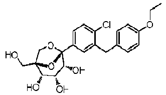 | Ertugliflozi n | (1S,2S,3R,4R,5S)-5-[ 4-chloro-3-[(4-ethoxy phenyl)methyl]phenyl ]-1-(hydroxymethyl)-6,8-dioxabicyclo[3 2. 1]octane-2,3,4-triol |
| | | | |
| 11 | 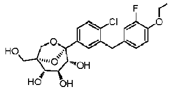 | Henagliflozi n | (1R,2S,3S,4R,5R)-5-[ 4-chloro-3-[(4-ethoxy -3-fluorophenyl)meth yl]phenyl]-1-(hydroxy methyl)-6,8-dioxabicy clo[3.2.1]octane-2,3,4 -triol |
| 12 | 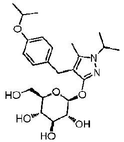 | Remoglifloz in | (2R,3S,4S,5R,6S)-2-( hydroxymethyl)-6-[5-methyl-1-propan-2-yl-4-[(4-propan-2-yloxy phenyl)methyl]pyrazo 1-3-yl]oxyoxane-3,4,5 -triol |
| 13 | 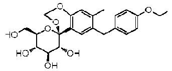 | / | (2'R,3'R,4'S,5'S,6'R)-6 -(4-ethoxybenzyl)-6'-( hydroxymethyl)-7-me thyl-3',4',5',6'-tetrahyd rospiro[benzo[d][1,3] dioxine-4,2'-pyran]-3', 4',5'-triol |
| 14 | 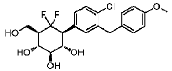 | Wangelie jing | (1R,2R,3S,4S,6R)-4-( 4-chloro-3-(4-ethoxyb enzyl)phenyl)-5,5-difl uoro-6-(hydroxymeth yl)cyclohexane-1,2,3-triol |
| 15 | 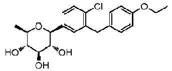 | Tianagliflo zin | (2S,3R,4S,5S,6R)-2-( 4-chloro-3-(4-ethoxyb enzyl)phenyl)-6-meth yltetrahydro-2H-pyra n-3,4,5-triol |
| 16 | 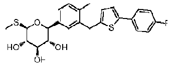 | Alliglifloz in | (2S,3R,4R,5S,6R)-2-( 3-((5-(4-fluorophenyl) thiophen-2-yl)methyl) -4-methylphenyl)-6-(methylthio)tetrahydro -2H-pyran-3,4,5-triol |

In the present invention, preferably, the SGLT-2 inhibitor-sarcosine cocrystal is in a fully crystalline form.

The cocrystals formed by the SGLT-2 inhibitors shown in the above Table 1 and sarcosine are commonly named as: dapagliflozin-sarcosine cocrystal, empagliflozin-sarcosine cocrystal, canagliflozin-sarcosine cocrystal, tofogliflozin-sarcosine cocrystal, ipragliflozin-sarcosine cocrystal, luseogliflozin-sarcosine cocrystal, sotagliflozin-sarcosine cocrystal, janagliflozin-sarcosine cocrystal, bexagliflozin-sarcosine cocrystal, alligliflozin-sarcosine cocrystal, henagliflozin-sarcosine cocrystal, ertugliflozin-sarcosine cocrystal, remogliflozin-sarcosine cocrystal and wangeliejing-sarcosine cocrystal etc., respectively.

In the present invention, a molar ratio of the SGLT-2 inhibitor to sarcosine in the SGLT-2 inhibitor-sarcosine cocrystal is preferably 1:(0.5-5); in some specific embodiments of the present invention, a molar ratio of the SGLT-2 inhibitor to sarcosine is preferably 1:0.6, 1:0.7, 1:0.8, 1:0.9, 1:0.95, 1:1.0, 1:1.05, 1:1.1, 1:1.2, 1:1.3, 1:1.4, 1:1.5, 1:1.6, 1:1.7, 1:1.8, 1:1.9 or 1:2.0, or a value in the range with the above ratio as upper limit or lower limit; more preferably, a molar ratio of the SGLT-2 inhibitor to sarcosine is 1:0.8, 1:0.9, 1:0.95, 1:1.0, 1:1.05, 1:1.1, 1:1.2, 1:1.3, 1:1.4 or 1:1.5.

The ligand of the SGLT-2 inhibitor-sarcosine cocrystal of the present invention is sarcosine. Sarcosine is a sports nutrition supplement. As a food additive or a sports nutrition supplement, sarcosine has a maximum daily dosage for adults of 2 grams or more. Sarcosine is a metabolite of glycine in the human body, and is widely distributed in muscles and other tissues of the human body.

Food-grade sarcosine is cheap and its quality is controllable. Sarcosine has a chemical formula of C₃H₇NO₂ and CAS number of 107-97-1, and its structural formula is as follows:

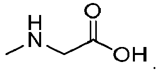

Therefore, the prepared SGLT-2 inhibitor-sarcosine cocrystal has high safety and low cost.

In the present invention, preferably, the SGLT-2 inhibitor-sarcosine cocrystal has a structure represented by formula I:

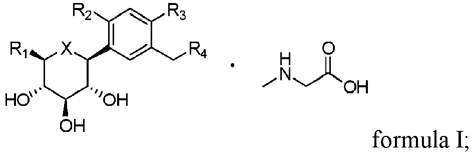

wherein, R₁, R₂, R₃, R₄ and X are respectively selected from the structures shown in the following Table 2:

**Table 2**

| | Dapagliflozi n (1) | Empagliflozi n (2) | Janagliflozin (3) | Bexagliflozi n (4) | Canagliflozi n (5) |
|---|---|---|---|---|---|
| R₁ | 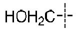 | 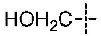 | 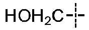 | 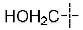 | 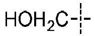 |
| R₂ | 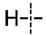 | 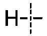 | 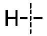 | 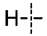 | 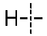 |
| R₃ | 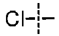 | 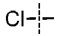 | 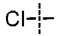 | 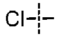 | 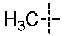 |
| R₄ | 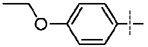 | 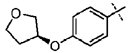 | 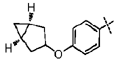 | 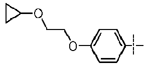 | 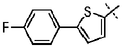 |
| X | O | O | O | O | O |

| | Ipragliflozin (6) | Tianagliflozi n (7) | Sotagliflozin (8) | Alligliflozin (9) | Luseogliflozi n (10) |
|---|---|---|---|---|---|
| R₁ | 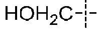 | 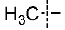 | 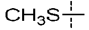 | 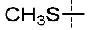 | 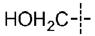 |
| R₂ | 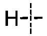 | 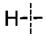 | 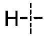 | 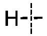 | 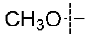 |
| R₃ | 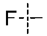 | 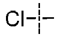 | 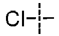 | 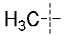 | 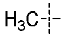 |
| R₄ | 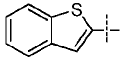 | 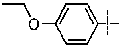 | 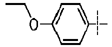 | 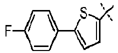 | 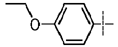 |
| X | O | O | O | O | S |

In the above Table 2, the dotted line indicates the positions of bonding.

Further preferably, the SGLT-2 inhibitor-sarcosine cocrystal has a structure represented by formula II: 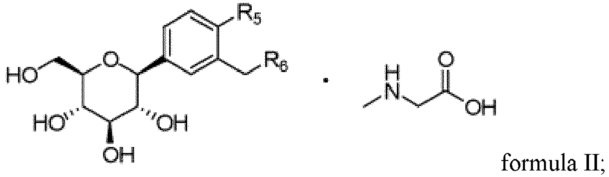 wherein, R₅ and R₆ are respectively selected from the structures shown in the following Table 3:

**Table 3**

| \ | Dapagliflozin | Empagliflozin | Canagliflozin | Janagliflozin | Bexagliflozin | Ipragliflozin |
|---|---|---|---|---|---|---|
| R₅ | 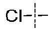 | 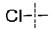 | 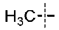 | 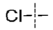 | 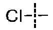 | 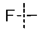 |
| R₆ | 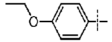 | 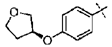 | 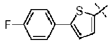 | 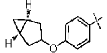 | 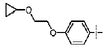 | 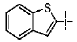 |

The SGLT-2 inhibitor-sarcosine cocrystal of the present invention may be a solvate or a hydrate of the cocrystal or a hydrate of the solvate of the cocrystal. The cocrystal of SGLT-2 and sarcosine can be characterized by diffraction angle 20 of the characteristic diffraction peak at a specific position in X-ray powder diffraction (XRPD) pattern. The XRPD instrument model used is: RigakuD/max-RB, the test conditions are: CuK α light source, 40kV voltage, 100mA current, slit 1°, 0.3mm, and the collecting software is: LJ51. Cu-K α radiation is used to perform detection.

The cocrystal of the SGLT-2 inhibitor and sarcosine of the present invention exhibits an XRPD spectrum having characteristic diffraction peaks at 10.6±0.2°, 19.6±0.2°, 22.1±0.2°, and 33.6±0.2° in addition to the characteristic diffraction peaks of the cocrystal portion of the SGLT-2 inhibitor.

In some specific embodiments of the present invention, the dapagliflozin-sarcosine cocrystal exhibits an XRPD spectrum having characteristic diffraction peaks at 3.8±0.2°, 10.6±0.2°, 13.7±0.2°, 17.0±0.2°, 18.0±0.2°, 18.6±0.2°, 19.6±0.2°, 20.1±0.2°, 21.4±0.2°, 22.1±0.2°, 23.0±0.2°, 25.4±0.2°, 27.6±0.2°, and 33.6± 0.2°.

More preferably, the dapagliflozin-sarcosine cocrystal exhibits an X-ray powder diffraction (XRPD) spectrum represented by diffraction angle 20 using Cu-Kα radiation having characteristic diffraction peaks at 3.77±0.2°, 10.66±0.2°, 11.21 +0.2°, 13.67±0.2°, 14.94±0.2°, 16.96±0.2°, 17.98±0.2°, 18.60±0.2°, 19.59±0.2°, 20.10±0.2°, 20.34±0.2°, 21.40±0.2°, 22.10±0.2°, 22.46±0.2°, 22.96±0.2°, 24.87±0.2°, 25.22±0.2°, 25.48±0.2°, 26.23±0.2°, 27.61±0.2°, 28.48±0.2°, and 33.62±0.2°.

In some specific embodiments of the present invention, the canagliflozin-sarcosine cocrystal exhibits an XRPD spectrum having characteristic diffraction peaks at 3.6±0.2°, 7.1±0.2°, 10.6±0.2°, 14.1±0.2°, 16.8±0.2°, 17.3±0.2°, 18.3±0.2°, 18.8±0.2°, 19.6±0.2°, 20.3±0.2°, 21.1±0.2°, 22.1±0.2°, 22.9±0.2°, 25.4± 0.2°, 28.2±0.2°, and 33.6±0.2°.

More preferably, the canagliflozin-sarcosine cocrystal exhibits an X-ray powder diffraction (XRPD) spectrum represented by diffraction angle 20 using Cu-Kα radiation having characteristic diffraction peaks at 3.63±0.2°, 3.63±0.2°, 7.10±0.2°, 10.60±0.2°, 14.08±0.2°, 15.93±0.2°, 16.77±0.2°, 17.34±0.2°, 18.32±0.2°, 18.79±0.2°, 19.60±0.2°, 20.30±0.2°, 20.60±0.2°, 21.11±0.2°, 22.06±0.2°, 22.89±0.2°, 25.41±0.2°, 28.29±0.2°, and 33.46±0.2°

The present invention employs infrared absorption spectroscopy to characterize the structure of the SGLT-2 inhibitor-sarcosine cocrystal, for example, the KBr pellet method is used to measure the infrared spectrum of the SGLT-2 inhibitor-sarcosine cocrystal. The instrument model of the infrared spectrometer used is: Thermo Nicolet 6700 FT-IR Spectrometer, and the test conditions are: KBr pellet method with a scanning range of 450-4000 cm-1. Characterization is performed by characteristic absorption peaks of the infrared spectrum.

The SGLT-2 inhibitor-sarcosine cocrystal of the present invention exhibits an infrared spectrum having characteristic absorption peaks at least at 3540±10 cm⁻¹, 2691±5 cm⁻¹, 2603±5 cm⁻¹, and 2420±5 cm⁻¹.

In some specific embodiments of the present invention, the dapagliflozin-sarcosine cocrystal exhibits an infrared spectrum having characteristic absorption peaks at 3543.67±10 cm⁻¹, 3161.94±10 cm⁻¹, 2690.95±5 cm⁻¹, 2603.78±5 cm⁻¹, 2419.39±5 cm⁻¹, 2360.665 cm⁻¹, 1599.61±5 cm⁻¹, 1510.92±5 cm⁻¹, 1291.19±5 cm⁻¹, and 1045.97 cm⁻¹.

In some specific embodiments of the present invention, the canagliflozin-sarcosine cocrystal exhibits an infrared spectrum having characteristic absorption peaks at least at 3543.82±10 cm⁻¹, 3153.82±10 cm⁻¹, 2690.68±5 cm⁻¹, 2603.06±5 cm⁻¹, 2419.53±5 cm⁻¹, 1596.33±5 cm⁻¹, 1507.48±5 cm⁻¹, 1086.11±5 cm⁻¹, 1062.19±5 cm⁻¹, and 829.61±5 cm⁻¹.

In the present invention, the SGLT-2 inhibitor-sarcosine cocrystal is characterized by differential scanning calorimetry. The instrument model used is: SII-DSC6220, and the parameters of the analysis method are: temperature range: 30°C-250°C, scan rate: 10°C/min, protective gas: nitrogen, 50 ml/min. The obtained differential scanning calorimetry (DSC) spectrum has an obvious endothermic peak at above 100°C, preferably at above 120°C.

The differential scanning calorimetry (DSC) spectrum data show that the dapagliflozin-sarcosine cocrystal provided by the present invention has a melting point of about 149.0°C, which is higher than that of the marketed dapagliflozin (S)-propylene glycol monohydrate, 70°C. An appropriate melting point makes the preparation less likely to melt and agglomerate during tablet granulation and tablet compression, which otherwise leads to sticking or unqualified uniformity. The cocrystal prepared by the present invention makes it more convenient to simplify the production process of the preparation and reduce the manufacturing cost.

The SGLT-2 inhibitor-sarcosine cocrystal provided by the present invention is characterized by thermogravimetric analysis. The instrument model of the thermogravimetric analyzer used is: SII-TG/DTA6200, and the parameters of the analysis method are: temperature ranges: 30°C-350°C, scan rate: 10°C/min, protective gas: nitrogen, 200 ml/min. Thermogravimetric analysis (TGA) spectrum shows no measurable weight loss below 120°C; and TGA-DTA spectrum shows a broad endothermic peak at 190°C to 230°C.

The structure of the SGLT-2 inhibitor-sarcosine cocrystal is also characterized by hydrogen nuclear magnetic resonance spectrum in the present invention. The instrument model used is: BrukerAVANCE600, resonance frequency: 600MHz, and the solvent used is deuterated methanol. The results show that the hydrogen nuclear magnetic resonance spectrum (HNMR) shows a -CH₃ resonance peak in a chemical shift range of 2.4 ppm to 3.2 ppm and a -CH₂- peak in a chemical shift range of 3.0 ppm to 4.0 ppm in addition to the resonance peak of the SGLT-2 inhibitor. The -CH₂- peak may or may not coincide with other resonance peaks of the SGLT-2 structure, indicating the presence of sarcosine.

The present invention provides a method for preparing the above-mentioned SGLT-2 inhibitor-sarcosine cocrystal, comprising:
mixing a solution of an SGLT-2 inhibitor with a solution of sarcosine, performing static crystallization or cooling crystallization, and performing solid-liquid separation to obtain the SGLT-2 inhibitor-sarcosine cocrystal.

In the present invention, the source of the SGLT-2 inhibitor is not particularly limited, and it may be commercially available or prepared according to methods well known to those skilled in the art. It may be a pure product, a crude product or an intermediate.

In the present invention, preferably, the solvent in the solution of the SGLT-2 inhibitor is selected from the group consisting of C1-C10 alcohol, C3-C10 ketone, ether, nitrile, and a combination thereof. More preferably, the solvent is selected from the group consisting of ethanol, acetone, tetrahydrofuran, acetonitrile, and a combination thereof. Further preferably, the solvent is ethanol.

The solvent in the solution of sarcosine is preferably water.

The molar ratio of SGLT-2 inhibitor to sarcosine is preferably 1:(0.5-5.0), more preferably 1:0.6, 1:0.7, 1:0.8, 1:0.9, 1:0.95, 1:1.0, 1:1.05, 1:1.1, 1:1.2, 1:1.3, 1:1.4, 1:1.5, 1:1.6, 1:1.7, 1:1.8, 1:1.9 or 1:2.0.

In the process of preparing the cocrystal of the present invention, the SGLT-2 inhibitor and sarcosine are added strictly according to the molar ratio of the two in the cocrystal, so that the SGLT-2 inhibitor and sarcosine properly form the cocrystal with no excess SGLT-2 inhibitor or sarcosine in the system.

In the present invention, after mixing the solution of the SGLT-2 inhibitor with the solution of sarcosine, heating can be carried out if the solution is not clarified.

In the present invention, preferably, the static crystallization or the cooling crystallization is preferably carried out at -20°C to 40°C, and in some embodiments, the crystallization is preferably carried out at -15°C to 35°C, -10°C to 30°C, -5°C to 30°C, 0°C to 30°C, 5°C to 30°C, 10°C to 30°C, 15°C to 30°C, or 20°C to 30°C.

In the present invention, the static crystallization or the cooling crystallization is preferably carried out for 4 hours to 48 hours, more preferably 4 hours to 24 hours, 4 hours to 16 hours, 4 hours to 12 hours, and more preferably 8 hours to 12 hours.

In the present invention, after the SGLT-2 inhibitor-sarcosine cocrystal is obtained, it is preferably dried at preferably 20°C to 80°C, more preferably 30°C to 80°C.

The obtained SGLT-2 inhibitor-sarcosine cocrystal may be a solvate of the cocrystal, a hydrate of the solvate of the cocrystal, or a hydrate of the cocrystal.

The method for preparing the above-mentioned SGLT-2 inhibitor-sarcosine cocrystal provided by the present invention has a simple process, is easy to realize, has a short cycle, and can obtain a crystal form that is stable and will not be affected by crystallization conditions, storage conditions or environment to cause crystal transformation or mixed crystal phenomena.

The SGLT-2 inhibitor-sarcosine cocrystal prepared by the present invention is free of a single impurity exceeding 0.1%, and a total amount of impurities is less than 0.5%.

In the present invention, the crude product (or intermediate) of the SGLT-2 inhibitor is mixed with sarcosine, stirred at room temperature, and subjected to solid-liquid separation to obtain the complex of the SGLT-2 inhibitor and sarcosine, which has a remarkable purification effect on the crude product of the SGLT-2 inhibitor. After purification, the purity of the SGLT-2 inhibitor is not lower than 99%.

Since most of the chemical structures of the SGLT-2 inhibitors have classical glucoside structures or glucoside structure derivatives, the corresponding isomer impurities, ring-opening impurities, five-membered ring impurities or dimer impurities are inevitably produced in the preparation process. Therefore, the ability of salt formation, cocrystal formation, solvate or hydrate to remove these specific process impurities, non-specific process impurities, and process by-products has a great influence on the preparation yield of active ingredients, production cycle and production cost.

For example, a diastereomer impuritiy is produced in the preparation process, and the reaction equation is as follows: 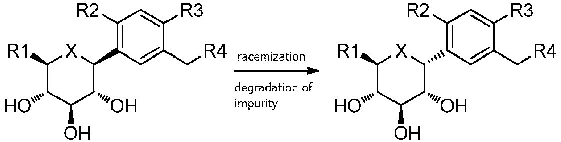 impurity 1.

Alternatively, the following impurity 2 (five-membered ring impurity) will also be produced in the preparation process of the above-mentioned SGLT-2 inhibitor: 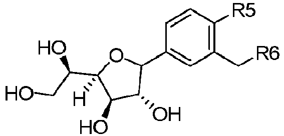 impurity 2.

The reaction mechanism of the above-mentioned five-membered ring impurity is:

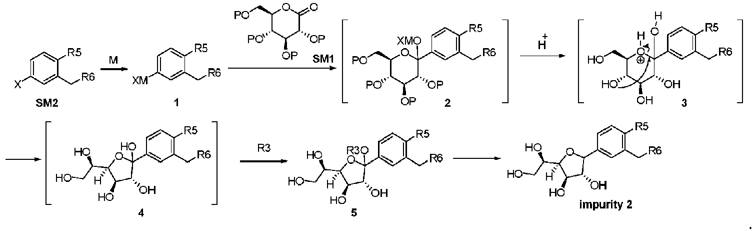

Alternatively, the following impurity 3 (ring-opening impurity) will also be produced in the preparation process of the above-mentioned SGLT-2 inhibitor: 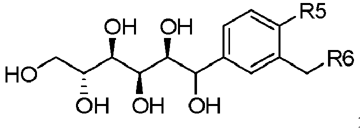 impurity 3.

The formation process of the ring-opening impurity is as follows:

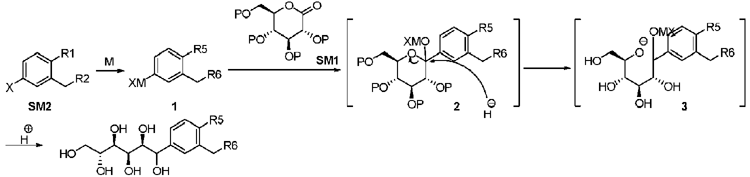

Alternatively, the dimer impurity 4 (a dimer impurity) will also be produced by the intermolecular polymerization reaction under severe reaction conditions in the preparation process of the above-mentioned SGLT-2 inhibitor.

The structure formula of the dimer impurity is as follows: 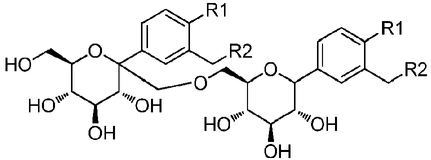 impurity 4

The formation process of above-mentioned dimer impurity is as follows:

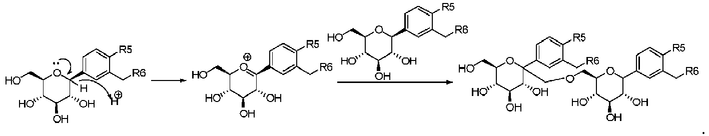

Wherein, X is halogen (bromine Br or iodine I); M is magnesium Mg; and P is a protecting group, such as tetramethylsilane TMS.

R₁ to R₅ are the same as above and will not be repeated here.

In addition to the specific process impurities (diastereoisomer impurities, five-membered ring impurities, ring-opening impurities and dimer impurities) related to the classic glucoside structure in the structure of the SGLT-2 inhibitor, a large number of non-specific impurities will also be produced during the process. The above-mentioned specific impurities and non-specific impurities in the intermediate or crude product of the SGLT-2 inhibitor are difficult to be removed to below the limit of impurities allowed for pharmaceutical use by one-time simple extraction or solvent recrystallization.

Surprisingly, by using the method of the present invention in which a complex of the crude product of the SGLT-2 inhibitor and sarcosine is formed, more than 95% of the impurities including process impurities, intermediates in the previous steps, and material residues contained in the crude product of the SGLT-2 inhibitor can be removed. The similar polarity and structure of these impurities to the target API make it difficult to achieve the purpose of purification by simple liquid-liquid extraction or solvent recrystallization. By forming a complex of the SGLT-2 inhibitor and sarcosine, the purity of the crude SGLT-2 inhibitor could be increased from 78% to 99% or more at one time, and the number of impurities and the total amount of impurities detected are significantly reduced. Or the obtained complex of the SGLT-2 inhibitor and sarcosine with high purity is subjected to simple dissociation to obtain a free-state SGLT-2 inhibitor with higher purity, which is then directly used as the target component or used to prepare other pharmaceutically acceptable crystal forms or cocrystals.

Specifically, the present invention provides a method for purifying an SGLT-2 inhibitor, comprising:

mixing the crude product of the SGLT-2 inhibitor with sarcosine, stirring at room temperature, and performing solid-liquid separation to obtain a complex of the SGLT-2 inhibitor and sarcosine.

In the present invention, preferably, the crude product of the SGLT-2 inhibitor is dissolved in a solvent, and the solvent is preferably selected from C1-C10 alcohol, C3-C10 ketone, ether, or nitrile, or a mixed solvent of the above-mentioned solvent and water, or a mixed solvent of the above-mentioned solvents. More preferably, the solvent is one or more of ethanol, acetone, tetrahydrofuran and acetonitrile, or one or more of ethanol, acetone, tetrahydrofuran and acetonitrile mixed with water; more preferably ethanol.

In the present invention, preferably, sarcosine is dissolved in a solvent, which is preferably water.

The molar ratio of the crude product of the SGLT-2 inhibitor to sarcosine is preferably 1:(0.5-5.0), and in some embodiments, the molar ratio of the crude product of the SGLT-2 inhibitor to sarcosine is preferably 1:0.7, 1:0.8, 1:0.9, 1:0.95, 1:1.0, 1:1.05, 1:1.1, 1:1.2, 1:1.3, 1:1.4, 1:1.5, 1:1.6, 1:1.7, 1:1.8, 1:1.9, 1:2.0, or 1:3.0; more preferably, the molar ratio of the crude product of the SGLT-2 inhibitor to sarcosine is 1:1.0, 1:1.1, 1:1.2, 1:1.3, 1:1.4, 1:1.5, 1:1.6, 1:1.7, 1:1.8, 1:1.9, 1:2.0, or 1:3.0.

In the present invention, during the purification process, the above-mentioned sarcosine is added in excess, and the resulting complex of the SGLT-2 inhibitor and sarcosine comprises the cocrystal formed by the SGLT-2 inhibitor and sarcosine, and some excess sarcosine.

In the present invention, preferably, the above solid-liquid separation is preferably performed by static crystallization or cooling crystallization.

In the present invention, preferably, the static crystallization or the cooling crystallization is preferably performed at -20°C to 40°C, more preferably -10°C to 40°C, 0°C to 40°C, 10°C to 40°C, 15°C to 40°C, 20°C to 40°C, 20°C to 35°C, or 20°C to 30°C.

In the present invention, preferably, the stirring is preferably performed for 6 hours to 16 hours, more preferably 6 hours to 14 hours, 7 hours to 13 hours, or 8 hours to 12 hours.

In the present invention, the obtained complex of the SGLT-2 inhibitor and sarcosine is subjected to solid-liquid separation to obtain a solid, which is then preferably dried at preferably 20°C to 80°C, more preferably 30°C to 80°C.

In the present invention, preferably, after obtaining the complex of the SGLT-2 inhibitor and sarcosine, the method further comprises:

dissociating the complex of the SGLT-2 inhibitor and sarcosine to obtain a pure product of the SGLT-2 inhibitor in free state.

In the present invention, preferably, the pure product of the SGLT-2 inhibitor in the free state has a HPLC normalized purity no less than 99%.

Then performing recrystallization or co-crystallization to prepare the final pharmaceutically acceptable crystal form of the SGLT-2 inhibitor.

In the present invention, preferably, the final pharmaceutically acceptable crystal form of the SGLT-2 inhibitor is selected from the group consisting of a pure product, solvate, hydrate, hydrate of solvate, co-crystal and double salt of the SGLT-2 inhibitor.

The present invention has no special limitation on the preparation process or source of the crude product of the SGLT-2 inhibitor, and it may be the crude product of the SGLT-2 inhibitor prepared by methods well known to those skilled in the art, such as the methods described in patent documents WO03099836A1 or CN100534997C. A content of the SGLT-2 inhibitor with the following structure formula 1 in the crude product or intermediate of the SGLT-2 inhibitor should not be less than 70%, preferably not less than 75%. 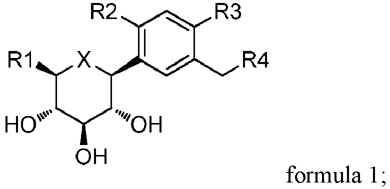 wherein, R1, R2, R3, R4 and X are listed in Table 2.

The dissociation method is not particularly limited in the present invention, and may be a method well known to those skilled in the art.

In some specific embodiments of the present invention, dissociation is performed by liquid-liquid extraction, and then the organic phase is collected and concentrated to obtain a pure product of the SGLT-2 inhibitor in the free state.

Experiment results show that the above-mentioned purification method provided by the present invention has a significant impurity removal effect on non-specific impurities, specific impurities, diastereomer impurities, five-membered ring impurities, ring-opening impurities and dimer impurities. The number of impurities is reduced by 90% or more, and the total amount of the impurities detected and the amount of the single impurity detected are both removed by 95% or more. The complex of the SGLT-2 inhibitor and sarcosine or the SGLT-2 inhibitor in the free state after purification has the number of impurities of 6 or less and a total content of impurities of 1.0% or less.

The above-mentioned purification method of the present invention can be used for the intermediate of the SGLT-2 inhibitor in the preparation process, and can also be used for the crude product before salt formation, cocrystal formation, and crystallization, preferably used for the intermediate or crude product with the intermediate of the SGLT-2 inhibitor that has same chemical structure as the target SGLT-2 inhibitor.

In the present invention, preferably, after obtaining the pure product of the SGLT-2 inhibitor in the free state, the pure product of the SGLT-2 inhibitor in the free state can be used as a raw material to directly prepare the final pharmaceutically acceptable crystal form of the SGLT-2 inhibitor;
alternatively, recrystallization or co-crystallization can be performed using the pure product of the SGLT-2 inhibitor in the free state as a raw material to prepare the final pharmaceutically acceptable crystal form of the SGLT-2 inhibitor.

In the present invention, the final pharmaceutically acceptable crystal form of the SGLT-2 inhibitor is preferably a pure product, solvate, hydrate, hydrate of solvate, co-crystal or double salt of the SGLT-2 inhibitor.

The present invention provides a pharmaceutical composition, comprising the above-mentioned SGLT-2 inhibitor-sarcosine cocrystal, the SGLT-2 inhibitor-sarcosine cocrystal prepared by the above preparation method, the pure product of the SGLT-2 inhibitor in the free state obtained by the above purification method, or the final pharmaceutically acceptable crystal form of the SGLT-2 inhibitor prepared by recrystallization or co-crystallization such as a pure product, solvate, hydrate, hydrate of solvate, cocrystal or double salt of the SGLT-2 inhibitor, and a pharmaceutically acceptable carrier, excipient, diluent, adjuvant, vehicle or a combination thereof.

When the active ingredient is administered in low doses, the particles of the active ingredient may be reduced to an appropriate size by physical means such as grinding or sieving in order to ensure uniform distribution of the active ingredient. The SGLT-2 inhibitor-sarcosine cocrystal of the invention has high stability, which is beneficial for uniform distribution in the product of a pharmaceutical composition when used as an active ingredient. Due to the high melting point, the cocrystal will not melt under heat, agglomerate or produce electrostatic adsorption during the crushing or grinding process, with no change in the crystal form during the grinding or crushing process, and the resulting final pharmaceutical composition has a uniform distribution of active ingredients and conforms to the labeled amount with high reproducibility.

The present invention provides use of the above-mentioned SGLT-2 inhibitor-sarcosine cocrystal, the SGLT-2 inhibitor-sarcosine cocrystal prepared by the above preparation method, the pure product of the SGLT-2 inhibitor in the free state obtained by the above purification method, the final pharmaceutically acceptable crystal form of the SGLT-2 inhibitor prepared by recrystallization or co-crystallization such as a pure product, solvate, hydrate, hydrate of solvate, cocrystal or double salt of the SGLT-2 inhibitor, or the above-mentioned pharmaceutical composition in the manufacture of a medicament for preventing, treating or alleviating diabetes or a complication thereof, a cardiovascular and cerebrovascular disease, or nephropathy caused by a non-diabetic disease.

The present invention provides use of the above-mentioned SGLT-2 inhibitor-sarcosine cocrystal, the SGLT-2 inhibitor-sarcosine cocrystal prepared by the above preparation method, the pure product of the SGLT-2 inhibitor in the free state obtained by the above purification method, the final pharmaceutically acceptable crystal form of the SGLT-2 inhibitor prepared by recrystallization or co-crystallization such as a pure product, solvate, hydrate, hydrate of solvate, cocrystal or double salt of the SGLT-2 inhibitor, or the above-mentioned pharmaceutical composition in the manufacture of a medicament for lowering blood pressure.

In the present invention, preferably, the diabetes and the complication thereof include but are not limited to:
one or more of essential hypertension, type 2 diabetes with hypertension, nephropathy with type 2 diabetes, nephropathy with hypertension and diabetes, nephropathy, type 1 diabetes, nephropathy with type 1 diabetic, liver fibrosis, insulin resistance, hyperglycemia, hyperinsulinemia, elevated fatty acid or glycerol level in blood, hyperlipidemia, dyslipidemia, and obesity.

The above-mentioned SGLT-2 inhibitor-sarcosine cocrystal, the pure product of the SGLT-2 inhibitor in the free state obtained by the above purification method, the final pharmaceutically acceptable crystal form of the SGLT-2 inhibitor prepared by recrystallization or co-crystallization such as a pure product, solvate, hydrate, hydrate of solvate, cocrystal or double salt of the SGLT-2 inhibitor, or the pharmaceutical composition, when used in the manufacture of a medicament for preventing, treating or alleviating diabetes and a complication thereof, or a cardiovascular and cerebrovascular disease, or used in the manufacture of a medicament for lowering blood pressure or nephropathy caused by non-diabetes, can be used alone or in combination with other drugs.

The present invention provides a method for preventing, treating or alleviating diabetes or a complication thereof, comprising contacting the above-mentioned SGLT-2 inhibitor-sarcosine cocrystal, the SGLT-2 inhibitor-sarcosine cocrystal prepared by the above preparation method, the pure product of the SGLT-2 inhibitor in the free state obtained by the above purification method, the final pharmaceutically acceptable crystal form of the SGLT-2 inhibitor prepared by recrystallization or co-crystallization such as a pure product, solvate, hydrate, hydrate of solvate, cocrystal or double salt of the SGLT-2 inhibitor, or the above-mentioned pharmaceutical composition with a biological specimen.

The present invention provides a method for lowering blood pressure, comprising contacting the above-mentioned SGLT-2 inhibitor-sarcosine cocrystal, the SGLT-2 inhibitor-sarcosine cocrystal prepared by the above preparation method, the pure product of the SGLT-2 inhibitor in the free state obtained by the above purification method, the final pharmaceutically acceptable crystal form of the SGLT-2 inhibitor prepared by recrystallization or co-crystallization such as a pure product, solvate, hydrate, hydrate of solvate, cocrystal or double salt of the SGLT-2 inhibitor, or the above-mentioned pharmaceutical composition with a biological specimen.

The present invention provides a method for treating nephropathy caused by non-diabetes, comprising contacting the above-mentioned SGLT-2 inhibitor-sarcosine cocrystal, the SGLT-2 inhibitor-sarcosine cocrystal prepared by the above preparation method, the pure product of the SGLT-2 inhibitor in the free state obtained by the above purification method, the final pharmaceutically acceptable crystal form of the SGLT-2 inhibitor prepared by recrystallization or co-crystallization such as a pure product, solvate, hydrate, hydrate of solvate, cocrystal or double salt of the SGLT-2 inhibitor, or the above-mentioned pharmaceutical composition with a biological specimen.

Compared with the prior art, the present invention provides an SGLT-2 inhibitor-sarcosine cocrystal with the following beneficial effects:
1. Using sarcosine as a ligand, it has high safety and low cost;
2. The pharmaceutical cocrystal has high stability, and its crystal form is not likely to change during the production or storage process of the preparation composition; It will not melt under heat, stick, agglomerate or generate static electricity during storage or preparation process at high temperature or the production of preparations, and thus a good mixing uniformity.
3. As an active ingredient, it is evenly distributed in the prepared pharmaceutical composition, such that the pharmaceutical composition has good in vivo release, absorption and pharmaceutical effect with small differences between batches;
4. The SGLT-2 inhibitor-sarcosine cocrystal does not change the solubility of drugs used as raw materials of oral solid preparation in physiological media with different pH, which is very important to ensure the absorption of oral solid preparations;
5. It has high physical and chemical stability, which is more convenient for storage and transportation;
6. The preparation process is simple, has high reproducibility, a short crystallization time, and a low requirement for process condition, and thus has high economic benefits;
7. The preparation process avoids the use of unsafe solvents;
8. During the preparation process of the SGLT-2 inhibitor, the final intermediate or crude product of the SGLT-2 inhibitor can be purified and dissociated by forming a complex with sarcosine to obtain a SGLT-2 inhibitor in free state with a purity of 99% or more, followed by subsequent recrystallization or co-crystallization, or co-crystallization with sarcosine according to the target API without additional cumbersome impurity removal steps.

### BRIEF DESCRIPTION OF DRAWINGS

Figure 1 shows the X-ray powder diffraction (XRPD) pattern and the single crystal diffraction pattern of the dapagliflozin-sarcosine cocrystal prepared in Example 1;
Figure 2 shows the infrared spectrum (IR) of the dapagliflozin-sarcosine cocrystal prepared in Example 1;
Figure 3 shows the differential scanning calorimetry (DSC) curve of the dapagliflozin-sarcosine cocrystal prepared in Example 1;
Figure 4 shows the thermogravimetric analysis (TGA) curve of the dapagliflozin-sarcosine cocrystal prepared in Example 1;
Figure 5 shows the hydrogen nuclear magnetic resonance (H-NMR) spectrum of the dapagliflozin-sarcosine cocrystal prepared in Example 1;
Figure 6 shows the X-ray powder diffraction (XRPD) pattern of the canagliflozin-sarcosine cocrystal prepared in Example 12;
Figure 7 shows the infrared spectrum (IR) of the canagliflozin-sarcosine cocrystal prepared in Example 12;
Figure 8 shows the differential scanning calorimetry (DSC) curve of the canagliflozin-sarcosine cocrystal prepared in Example 12;
Figure 9 shows the thermogravimetric analysis (TGA) curve of the canagliflozin-sarcosine cocrystal prepared in Example 12;
Figure 10 shows the hydrogen nuclear magnetic resonance (H-NMR) spectrum of the canagliflozin-sarcosine cocrystal prepared in Example 12;
Figure 11 shows an ellipsoid diagram of the crystal molecular structure of the dapagliflozin-sarcosine cocrystal.

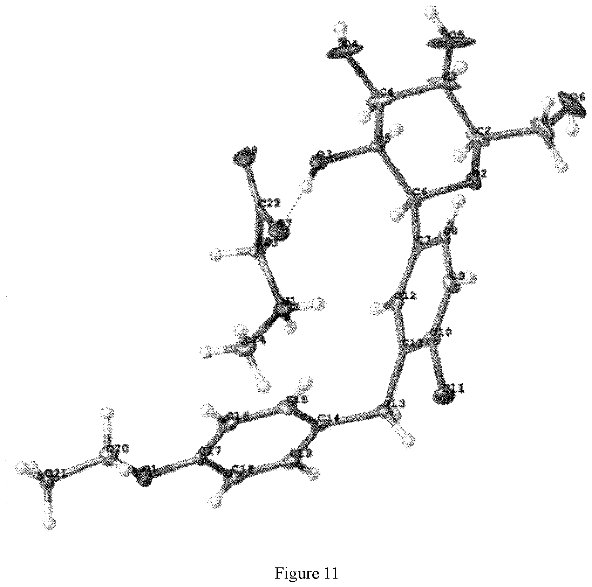

Figure 11 shows the ellipsoidal molecular structure of the single crystal structure of the dapagliflozin-sarcosine cocrystal.

### DETAILED DESCRIPTION

The raw material sarcosine used in the present invention is commercially available.

The raw material dapagliflozin used in the present invention is commercially available, or prepared according to known methods such as the method described in the patent document CN100534997 C.

The solvent used in the present invention is not particularly limited, and may be commercially available conventional solvents.

The instruments and methods used to collect data are as follows:
For collecting X-ray powder diffraction (XRPD) data, the instrument model used: RigakuD/max-RB, the test conditions: CuKα light source, 40kV voltage, 100mA current, slit 1°, 1°, 0.3 mm, collecting software: LJ51.

The instrument for single crystal X-ray diffraction: Gemini E single crystal diffractometer, crystal analysis software: Shelx97; CuKa as X-ray light source.

For collecting Infrared spectroscopy (IR) data, the instrument model used: Thermo Nicolet 6700 FT-IR Spectrometer, the test conditions: KBr pellet method, scanning range of 450 cm⁻¹ to 4000 cm⁻¹.

For collecting differential scanning calorimetry (DSC) data, the instrument model used: SII-DSC6220, parameters of analysis method: temperature range: 30°C to 250°C, scan rate: 10°C/min, protective gas: nitrogen, 50 ml/min.

For collecting thermogravimetric analysis (TGA) data, the instrument model used: SII-TG/DTA6200, parameters of analysis method: temperature range: 30°C to 350°C, scan rate: 10°C/min, protective gas: nitrogen, 200 ml/min.

For collecting hydrogen nuclear magnetic resonance (HNMR) data, the instrument model used: BrukerAVANCE600, the resonance frequency: 600MHz, and the solvent used: deuterated methanol.

The test conditions of liquid chromatography involved in the present invention: chromatographic column: Kromasil KR100-5-C18, 4.6×250 mm; mobile phase A: 0.1% phosphoric acid; mobile phase B: acetonitrile; detection wavelength: 220 nm; flow rate: 0.8 mL/min; injection volume: 20 µL; column temperature: 35°C. The conditions of liquid chromatography are shown in Table 4 below:

**Table 4 Test conditions of liquid chromatography**

| | | | | | | | | |
|---|---|---|---|---|---|---|---|---|
| t(min) | 0 | 25 | 30 | 35 | 40 | 45 | 45.1 | 60 |
| B(%) | 30 | 45 | 50 | 70 | 70 | 90 | 30 | 30 |

It should be noted that for the values or the numerical endpoints of the range involved in the technical solutions of the present invention, the meaning or scope of protection is not limited to the numbers themselves, and those skilled in the art can understand that they include those that have been widely accepted permissible error ranges in the art, such as experimental errors, measurement errors, statistical errors and random errors, etc., which are all included within the scope of the present invention.

In order to further illustrate the present invention, the SGLT-2 inhibitor-sarcosine cocrystal provided by the present invention and its preparation method and application are described in detail below in conjunction with the examples, but the protection scope of the present invention is not limited thereto.

In the following examples, all reagents including canagliflozin, sarcosine, etc. are commercially available unless otherwise stated.

Dapagliflozin used in the following examples was prepared according to the patent document WO03099836A1.

### Example 1. Preparation of dapagliflozin-sarcosine cocrystal

500 mL of absolute ethanol was added to a 1000 mL three-neck flask, and stirring was started. 100 g of dapagliflozin (HPLC normalized purity≥95%) prepared according to the method provided in patent document WO03099836A1 was added thereto and stirred until the solution was clarified. 30 mL of purified water was added to another 100 mL three-neck flask, and stirring was started. 21.80 g of sarcosine was added thereto and stirred until the solution was clarified. Then the resulting solution was added to the above solution of dapagliflozin in ethanol. The system was kept at 20°C to 30°C under stirring for 5 hours of crystallization, and then filtered. The collected solid was dried at 30°C to 40°C under vacuum for 6 hours to obtain 113 g of white solid with an HPLC normalized purity of 99.93%, which was free of impurity whose normalized content exceeded 0.1%.

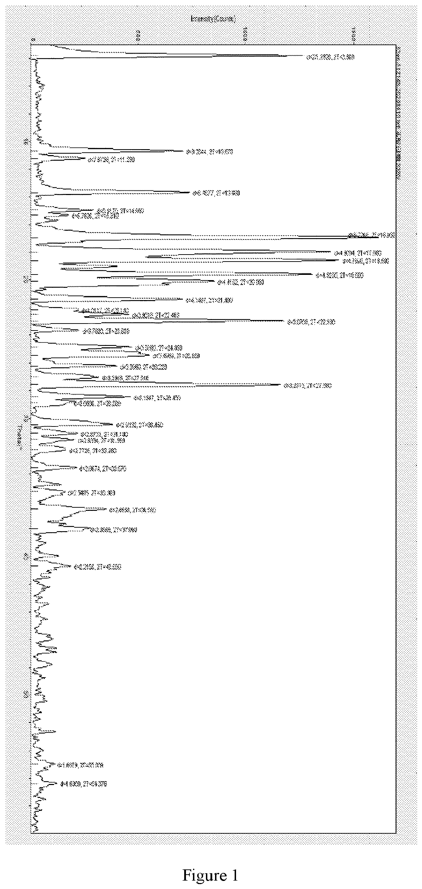

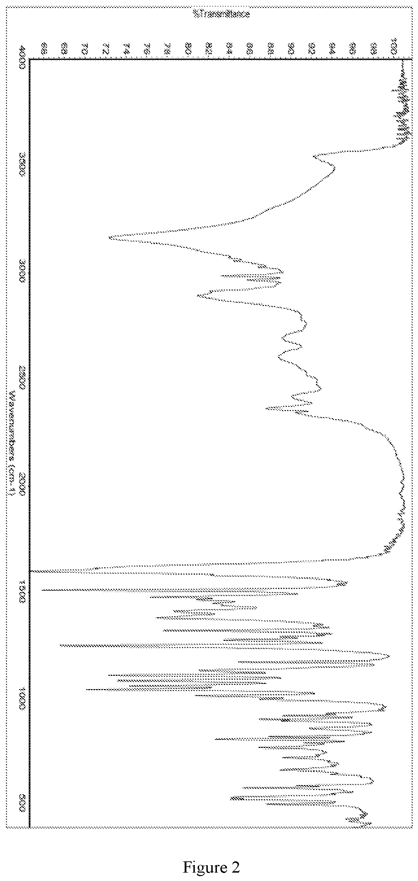

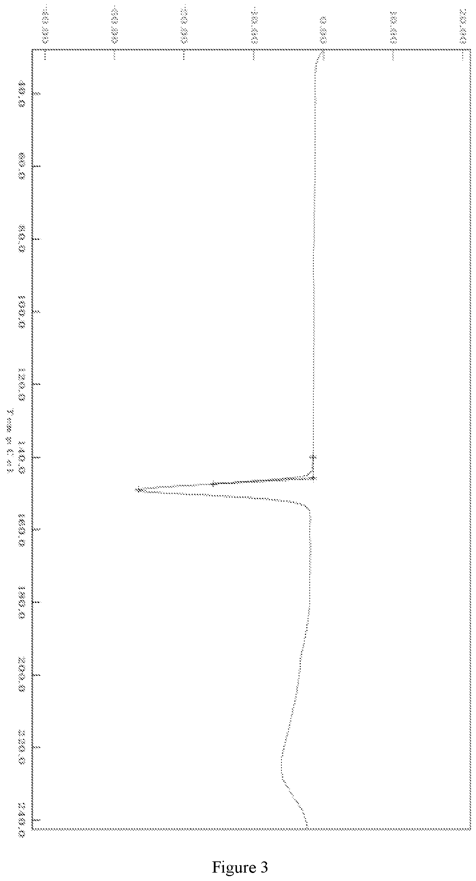

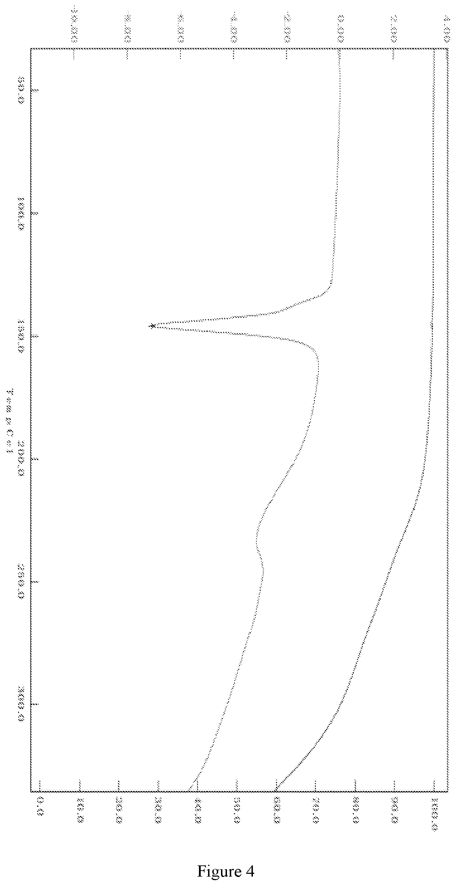

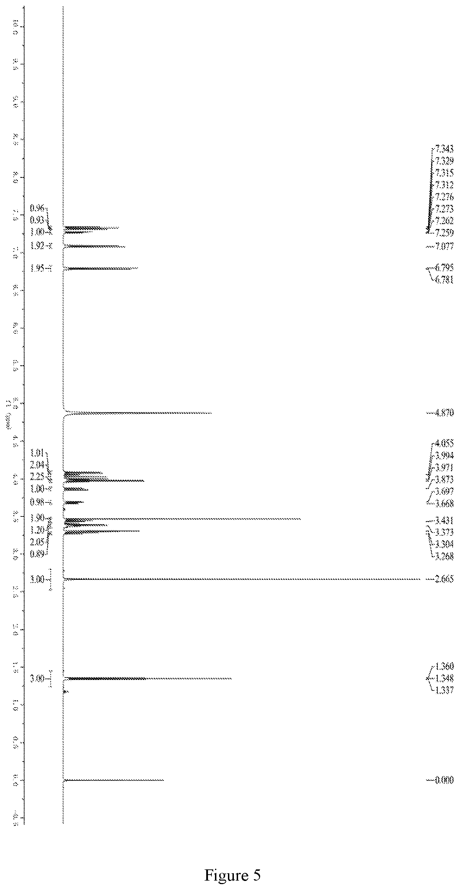

A dapagliflozin-sarcosine cocrystal was confirmed through X-ray powder diffraction (XRPD), infrared spectroscopy (IR), differential scanning calorimetry (DSC), thermogravimetric analysis (TGA), and hydrogen nuclear magnetic resonance (H-NMR), see Figures 1 to 5 for details.

¹HNMR (600MHz, MeOD) δ 7.343-7.329 (d, 1H, J=8.4Hz), 7.315-7.312 (d, 1H, J=1.8Hz) 7.276-7.259 (dd, 1H, J=8.4, 1.8Hz) , 7.091-7.077 (d, 2H, J=8.4Hz), 6.795-6.781 (d, 2H, J=8.4Hz), 4.089-4.073 (d, 1H, J=9.6Hz), 4.055-3.977 (q, 2H, J=15.0Hz) , 3.994-3.959 (q, 2H, J=7.2Hz) , 3.876-3.854 (dd, 1H, J=12.0, 1.8Hz) , 3.697-3.668 (dd, 1H, J=12.0, 5.4Hz) , 3.467 (s, 2H) 3.461-3.431 (m, 1H) , 3.409-3.370 (m, 2H) , 3.283-3.268 (m, 1H) , 2.665 (s, 3H) , 1.360-1.337 (t, 3H, J=7.2Hz)

It can be seen that the molar ratio of dapagliflozin to sarcosine is 1:1 according to the ¹HNMR spectrum data.

The dapagliflozin-sarcosine cocrystal exhibits an X-ray powder diffraction (XRPD) spectrum represented by diffraction angle 20 using Cu-Kα radiation having characteristic peaks and the respective intensities as shown in the following table 5, where the diffraction angle 20 has an error of 0.2°:

**Table 5. Characteristic peaks and respective intensities of dapagliflozin-sarcosine cocrystal in XRPD spectrum**

| No. | diffraction angle 2θ | D | I/Io | No. | diffraction angle 2θ | D | I/Io |
|---|---|---|---|---|---|---|---|
| 1 | 3.765 | 23.4510 | 72.3 | 10 | 20.343 | 4.3618 | 57.3 |
| 2 | 10.656 | 8.2953 | 40.8 | 11 | 21.404 | 4.1479 | 32.2 |
| 3 | 11.210 | 7.8862 | 16.4 | 12 | 22.096 | 4.0196 | 16.7 |
| 4 | 13.674 | 6.4705 | 51.0 | 12 | 22.962 | 3.8699 | 100 |
| 5 | 14.939 | 5.9253 | 17.7 | 13 | 25.482 | 3.4927 | 29.0 |
| 6 | 16.956 | 5.2247 | 44.8 | 14 | 27.606 | 3.2285 | 60.6 |
| 7 | 17.977 | 4.9301 | 89.6 | 15 | 30.435 | 2.9346 | 15.3 |
| 8 | 18.602 | 4.7658 | 51.6 | 16 | 33.617 | 2.6637 | 12.6- |
| 9 | 19.588 | 4.5283 | 95.2 | -- | -- | -- | -- |

The dapagliflozin-sarcosine cocrystal exhibits an infrared (IR) spectrum measured by the KBr pellet method having absorption peaks at 3543.67±10 cm⁻¹, 3161.94±10 cm⁻¹, 2888.82±5 cm⁻¹, 2690.95±5 cm⁻¹, 2603.78±5 cm⁻¹, 2419.39±5 cm⁻¹, 2360.66±5 cm⁻¹, 1599.61±5 cm⁻¹, 1510.92±5 cm⁻¹, 1291.19±5 cm⁻¹, 1045.97±5 cm⁻¹, and 812.44±5 cm⁻¹.

The dapagliflozin-sarcosine cocrystal exhibits a differential scanning calorimetry (DSC) spectrum having an endothermic peak in the range of 140.0°C to 155.0°C with a peak value of 149.0°C as melting temperature.

The dapagliflozin-sarcosine cocrystal exhibits a thermogravimetric analysis (TGA) spectrum having no measurable weight loss below 150°C and a TGA-DTA spectrum having a broad endothermic peak at 190°C to 230°C. The thermogravimetric analysis (TGA) spectrum and a water content of 0.10% shown by the moisture meter (instrument model: Metrohm 915-KF), indicate that the dapagliflozin-sarcosine cocrystal of the present invention exists in the form of no hydrate or no solvate.

### Example 2: Preparation of dapagliflozin-sarcosine cocrystal

50 mL of absolute ethanol was added to a 100 mL three-neck flask, and stirring was started. 10 g of dapagliflozin (HPLC normalized purity≥95%) prepared according to the method provided in patent document WO03099836A1 was added thereto and stirred until the solution was clarified. 2 mL of purified water was added to a 10 mL single-neck flask, and stirring was started. 1.10 g of sarcosine was added thereto and stirred until the solution was clarified. Then the resulting solution was added to the above solution of dapagliflozin in ethanol. The system was kept at 10°C to 20°C under stirring for 5 hours of crystallization, and then filtered. The collected solid was dried at 40°C to 50°C under vacuum for 4 hours to obtain 4.8 g of dapagliflozin-sarcosine cocrystal as a white solid with an HPLC normalized purity of 99.89%, which was free of impurity whose normalized content exceeded 0.1%.

### Example 3: Preparation of dapagliflozin-sarcosine cocrystal

50 mL of absolute ethanol was added to a 100 mL three-neck flask, and stirring was started. 10 g of dapagliflozin (HPLC normalized purity≥95%) prepared according to the method provided in patent document WO03099836A1 was added thereto and stirred until the solution was clarified. 5.0 mL of purified water was added to a 10 mL single-neck flask, and stirring was started. 3.27 g of sarcosine was added thereto and stirred until the solution was clarified. Then the resulting solution was added to the above solution of dapagliflozin in ethanol. The system was kept at -10°C to 10°C under stirring for 5 hours of crystallization, and then filtered. The collected solid was dried at 50°C to 60°C under vacuum for 4 hours to obtain 10.85 g of dapagliflozin-sarcosine cocrystal as a white solid with an HPLC normalized purity of 99.87%, which was free of impurity whose normalized content exceeded 0.1%.

### Example 4: Preparation of dapagliflozin-sarcosine cocrystal

50 mL of acetonitrile was added to a 100 mL three-neck flask, and stirring was started. 10 g of dapagliflozin (HPLC normalized purity≥95%) prepared according to the method provided in patent document WO03099836A1 was added thereto and stirred until the solution was clarified. 3.0 mL of purified water was added to a 10 mL single-neck flask, and stirring was started. 2.18 g of sarcosine was added thereto and stirred until the solution was clarified. Then the resulting solution was added to the above solution of dapagliflozin in acetonitrile. The system was kept at 30°C to 40°C under stirring for 5 hours of crystallization, and then filtered. The collected solid was dried at 70°C to 80°C under vacuum for 4 hours to obtain 9.70 g of dapagliflozin-sarcosine cocrystal as a white solid with an HPLC normalized purity of 99.90%, which was free of impurity whose normalized content exceeded 0.1%.

### Example 5: Preparation of dapagliflozin-sarcosine cocrystal

50 mL of tetrahydrofuran was added to a 100 mL three-neck flask, and stirring was started. 10 g of dapagliflozin (HPLC normalized purity≥95%) prepared according to the method provided in patent document WO03099836A1 was added thereto and stirred until the solution was clarified. 3.0 mL of purified water was added to a 10 mL single-neck flask, and stirring was started. 2.20 g of sarcosine was added thereto and stirred until the solution was clarified. Then the resulting solution was added to the above solution of dapagliflozin in tetrahydrofuran. The system was kept at 20°C to 30°C under stirring for 5 hours of crystallization, and then filtered. The collected solid was dried at 60°C to 70°C under vacuum for 4 hours to obtain 9.15 g of dapagliflozin-sarcosine cocrystal as a white solid with an HPLC normalized purity of 99.73%, which was free of impurity whose normalized content exceeded 0.1%.

### Example 6: Preparation of dapagliflozin-sarcosine cocrystal

50 mL of acetone was added to a 100 mL three-neck flask, and stirring was started. 10 g of dapagliflozin (HPLC normalized purity≥95%) prepared according to the method provided in patent document WO03099836A1 was added thereto and stirred until the solution was clarified. 3.0 mL of purified water was added to a 10 mL single-neck flask, and stirring was started. 2.10 g of sarcosine was added thereto and stirred until the solution was clarified. Then the resulting solution was added to the above solution of dapagliflozin in acetone. The system was kept at 20°C to 30°C under stirring for 5 hours of crystallization, and then filtered. The collected solid was dried at 20°C to 30°C under vacuum for 6 hours to obtain 10.13 g of dapagliflozin-sarcosine cocrystal as a white solid with an HPLC normalized purity of 99.82%, which was free of impurity whose normalized content exceeded 0.1%.

### Example 7: Preparation of dapagliflozin-sarcosine cocrystal

25 mL of acetone and 25 mL of absolute ethanol were added to a 100 mL three-neck flask, and stirring was started. 10 g of dapagliflozin (HPLC normalized purity≥95%) prepared according to the method provided in patent document WO03099836A1 was added thereto and stirred until the solution was clarified. 3.0 mL of purified water was added to a 10 mL single-neck flask, and stirring was started. 2.15 g of sarcosine was added thereto and stirred until the solution was clarified. Then the resulting solution was added to the above solution of dapagliflozin in acetone and absolute ethanol. The system was kept at 20°C to 30°C under stirring for 5 hours of crystallization, and then filtered. The collected solid was dried at 30°C to 40°C under vacuum for 6 hours to obtain 10.89 g of dapagliflozin-sarcosine cocrystal as a white solid with an HPLC normalized purity of 99.82%, which was free of impurity whose normalized content exceeded 0.1%.

### Example 8: Purification of crude product of dapagliflozin

150 mL of absolute ethanol was added to a 250 mL three-neck flask, and stirring was started. 35 g of a crude product of dapagliflozin (HPLC normalized purity of 78.04%) prepared according to the method provided in patent document WO03099836A1 was added thereto and stirred until the solution was clarified. 10 mL of purified water was added to a 50 mL single-neck flask, and stirring was started. 9.00 g of sarcosine was added thereto and stirred until the solution was clarified. Then the resulting solution was added to the above solution of dapagliflozin in absolute ethanol. The system was kept at 20°C to 30°C under stirring for 5 hours of crystallization, and then filtered. The collected solid was dried at 30°C to 40°C under vacuum for 6 hours to obtain 25.4 g of a complex of dapagliflozin and sarcosine as a white solid with an HPLC normalized purity of 99.37%.

To a 250 mL three-neck flask, 50 mL of purified water, 150 mL of methyl tert-butyl ether, and 25 g of the complex of dapagliflozin and sarcosine were added, and stirred until the solution was clarified. The organic phase was then collected, washed by 50 ml of purified water for 3 times, evaporated under reduced pressure to remove the solvent, and dried at 30°C to 40°C in vacuum for 6 hours to obtain 19.5 g of dapagliflozin as a white solid (HPLC normalized purity of 99.98%).

### Comparative Example 1:

600 ml of methyl tert-butyl ether was added to a 3000 mL three-neck flask, and stirring was started. 35 g of a crude product of dapagliflozin (HPLC normalized purity of 78.04%) prepared according to the method provided in patent document WO03099836A1 was added thereto and stirred until the solution was clarified. According to the method for preparing dapagliflozin (S)-propylene glycol monohydrate in Example 6 of patent document WO2008002824A1, 6.51g of (S)-1,2-propylene glycol, 1.54 g of purified water, and 0.35 g of a seed crystal of dapagliflozin (S)-propylene glycol monohydrate were added thereto. The system was kept at 20°C to 30°C under stirring for 5 hours of crystallization, added with 1200 mL of cyclohexane, and then filtered. The collected solid was dried at 20°C to 25°C under vacuum for 4 hours to obtain 25 g of dapagliflozin (S)-propylene glycol monohydrate as an off-white solid (HPLC normalized purity of 93.59%).

To a 250 mL three-neck flask, 50 mL of purified water, 150 mL of methyl tert-butyl ether, and 20 g of the obtained dapagliflozin (S)-propylene glycol monohydrate were added, and stirred until the solution was clarified. The organic phase was then separated, washed by 50 ml of purified water for 3 times, and evaporated under reduced pressure to remove the solvent to obtain 18 g of dapagliflozin as a pale yellow foamy solid (HPLC normalized purity 95.78%).

### Example 9: Purification of intermediate of dapagliflozin

150 mL of absolute ethanol was added to a 250 mL three-neck flask, and stirring was started. 30 g of a crude product of dapagliflozin (batch number 20200416-2, yellow foamy solid, the detected impurities before purification are shown in table 6 below) prepared according to the method provided in patent document WO03099836A1 was added thereto and stirred until the solution was clarified. 9 mL of purified water was added to a 50 mL single-neck flask, and stirring was started. 7.55 g of sarcosine was added thereto and stirred until the solution was clarified. Then the resulting solution was added to the above solution of dapagliflozin in absolute ethanol. The system was kept at 20°C to 30°C under stirring for 5 hours of crystallization, and then filtered. The collected solid was dried at 30°C to 40°C under vacuum for 6 hours to obtain 25 g of a complex of dapagliflozin and sarcosine as a white solid, batch number: 202000714-1. The detected impurities after purification are shown in table 6 below.

250 ml (calculated according to the complex of dapagliflozin and sarcosine) of purified water was added to a reaction flask, and stirring was started. Then the complex of dapagliflozin and sarcosine and 250 ml (calculated according to the complex of dapagliflozin and sarcosine) of methyl tert-butyl ether were added. The system was subjected to protection of nitrogen, stirred at 20°C to 30°C, left to stand, and separated to collect the organic phase. The organic phase was washed twice with 250 ml of purified water. After separation, the solvent of the organic phase was removed under reduced pressure to obtain a foamy solid product. The solid product was dissolved with 3 times the volume of methyl tert-butyl ether to clarification, and added to 150 ml n-heptane solution to precipitate a white solid. Filtration was performed under protection of nitrogen, and the filter cake was collected and then dried at 40°C under vacuum to obtain a pure product of dapagliflozin in free state (yield of 80%).

**Table 6**

| | Crude product of dapagliflozin | Example 9 | Comparative Example 2 | Comparative Example 3 |
|---|---|---|---|---|
| Purification method | / | Forming a cocrystal with sarcosine | (S)-1,2-propylene glycol hydrate | Forming a cocrystal with proline |
| Batch number | 20200416-2 | 20200714-1 | 20200714-4 | 20200418-1 |
| Property | Yellow foamy solid | White solid | White solid | White foamy solid |
| Yield (%) | / | 69 | 70 | 37 |
| HPLC normalized content (%) | 87.87 | 99.69 | 97.84 | 98.27 |
| Total content of impurities (%) | 12.13 | 0.31 | 2.16 | 1.72 |
| Number of impurities | 29 | 3 | 7 | 10 |
| Detected impurities | / | One impurity exceeding 0.1%, the detected content: 0.17% | 4 impurities exceeding 0.1%, maximal single impurity: 1.48% | 6 impurities exceeding 0.1%, maximal single impurity: 1.12% |
| Impurity removal effect | | 26 impurities were completely removed; the removal rate of ring-opening impurities and dimer impurities is 100%, and the removal rate of five-membered ring impurities and diastereomer impurities is over 98%. | 22 impurities were completely removed; the removal rate of ring-opening impurities is about 50%, and the removal rate of five-membered ring impurities and diastereomer impurities is 90%. | 19 impurities were completely removed; the removal rate of ring-opening impurities is 60%, the removal rate of dimer impurities is 100%, and the removal rate of five-membered ring impurities and diastereomer impurities is over 80%. |

### Comparative Example 2:

520 ml of methyl tert-butyl ether was added to a 3000 mL three-neck flask, and stirring was started. 30 g of a crude product of dapagliflozin (batch number 20200416-2, yellow foamy solid, the detected impurities before purification are shown in table 6) prepared according to the method provided in patent document WO03099836A1 was added thereto and stirred until the solution was clarified. According to the method for preparing dapagliflozin (S)-propylene glycol monohydrate in Example 6 of patent document WO2008002824A1, 5.58 g of (S)-1,2-propylene glycol, 1.32 g of purified water, and 0.30 g of a seed crystal of dapagliflozin (S)-propylene glycol monohydrate were added thereto. The system was kept at 20°C to 30°C under stirring for 8 hours of crystallization, added with 1030 mL of cyclohexane, and then filtered. The collected solid was dried at 20°C to 25°C under vacuum for 4 hours to obtain 23 g of dapagliflozin (S)-propylene glycol monohydrate as an off-white solid, batch number: 20200714-4. The detected impurities after purification are shown in table 6.

### Comparative Example 3:

170 ml of isopropanol was added to a 500 mL single-neck flask. Then 35 g of a crude product of dapagliflozin (HPLC: 87.87%) prepared according to the method provided in patent document WO03099836A1 was added thereto and stirred until the solution was clarified.

17 ml of purified water was added to a 1000 mL three-neck flask, and stirring was started. 19.71 g of L-proline was added. The system was heated to 80°C, added with 170 mL of isopropanol followed by the above-mentioned solution of dapagliflozin in isopropanol under rapid stirring, slowly cooled to room temperature, kept at 20°C to 30°C under stirring for 5 hours of crystallization, and then filtered. The filter cake was washed with 20 mL of isopropanol and 20 mL of n-hexane. The collected solid was dried at 30°C to 40°C in vacuum for 4 hours to obtain 33.21 g of dapagliflozin bis-L-proline cocrystal as an off-white solid (HPLC: 97.41%).

To a 250 mL three-neck flask, 50 mL of purified water, 150 mL of methyl tert-butyl ether, and 25 g of the obtained dapagliflozin bis-L-proline cocrystal were added, and stirred until the solution was clarified. The organic phase was then separated, washed by 50 ml of purified water for 3 times, and evaporated under reduced pressure to remove the solvent to obtain 14.8 g of dapagliflozin as an off-white foamy solid (HPLC: 98.27%).

### Example 10: Purification of crude product of dapagliflozin

150 mL of absolute ethanol was added to a 250 mL three-neck flask, and stirring was started. 30 g of a crude product of dapagliflozin (batch number 20200602, yellow foamy solid, the detected impurities before purification are shown in table 7 below) prepared according to the method provided in patent document WO03099836A1 was added thereto and stirred until the solution was clarified. 10 mL of purified water was added to a 50 mL single-neck flask, and stirring was started. 9.79 g of sarcosine was added thereto and stirred until the solution was clarified. Then the resulting solution was added to the above solution of dapagliflozin in absolute ethanol. The system was kept at 20°C to 30°C under stirring for 5 hours of crystallization, and then filtered. The collected solid was dried at 30°C to 40°C under vacuum for 6 hours to obtain 23 g of a complex of dapagliflozin and sarcosine as a white solid, batch number: 202000714-2. The detected impurities are shown in table 7 below.

**Table 7**

| | Crude product of dapagliflozin | Example 10 | Comparative Example 4 |
|---|---|---|---|
| Purification method | / | Forming a complex with sarcosine | Forming a solvate with (S)-1,2-propylene glycol/water |
| Batch number | 20200602 | 20200714-2 | 20200714-5 |
| Property | Yellow foamy solid | White solid | White solid |
| Yield (%) | / | 75 | 78 |
| HPLC normalized content (%) | 84.49 | 99.34 | 94.86 |
| Total content of impurities (%) | 15.51 | 0.66 | 5.14 |
| Number of impurities | 32 | 5 | 12 |
| Detected impurities | / | 2 impurities exceeding 0.1%, the detected content: 0.31% | 8 impurities exceeding 0.1%, maximal single impurity: 3.3% |
| Impurity removal effect | / | 27 impurities were completely removed; the removal rate of ring-opening impurities and dimer impurities is | 20 impurities were completely removed; the removal rate of ring-opening impurities is about 50%, and the |
| | | 100%, and the removal rate of five-membered ring impurities and isomer impurities is over 98%. | removal rate of five-membered ring impurities and isomer impurities is about 85%. |

### Comparative Example 4:

600 ml of methyl tert-butyl ether was added to a 3000 mL three-neck flask, and stirring was started. 30 g of a crude product of dapagliflozin (batch number 202000602, yellow foamy solid, the detected impurities before purification are shown in table 7) prepared according to the method provided in patent document WO03099836A1 was added thereto and stirred until the solution was clarified. According to the method for preparing dapagliflozin (S)-propylene glycol monohydrate in Example 6 of patent document WO2008002824A1, 5.58 g of (S)-1,2-propylene glycol, 1.54 g of purified water, and 0.30 g of a seed crystal of dapagliflozin (S)-propylene glycol monohydrate were added thereto. The system was kept at 20°C to 30°C under stirring for 8 hours of crystallization, added with 1030 mL of cyclohexane, and then filtered. The collected solid was dried at 20°C to 25°C under vacuum for 4 hours to obtain 24 g of dapagliflozin (S)-propylene glycol monohydrate as an off-white solid, batch number: 20200714-5. The detected impurities after purification are shown in table 7.

### Example 11: Purification of crude product of dapagliflozin

150 mL of absolute ethanol was added to a 250 mL three-neck flask, and stirring was started. 30 g of a crude product of dapagliflozin (batch number 20200703-3, yellow foamy solid, the detected impurities before purification are shown in table 8 below) prepared according to the method provided in patent document WO03099836A1 was added thereto and stirred until the solution was clarified. 10 mL of purified water was added to a 50 mL single-neck flask, and stirring was started. 9.8 g of sarcosine was added thereto and stirred until the solution was clarified. Then the resulting solution was added to the above solution of dapagliflozin in absolute ethanol. The system was kept at 20°C to 30°C under stirring for 5 hours of crystallization, and then filtered. The collected solid was dried at 30°C to 40°C under vacuum for 6 hours to obtain 21 g of a complex of dapagliflozin and sarcosine as a white solid, batch number: 20200714-3. The detected impurities after purification are shown in table 8 below.

**Table 8**

| | Crude product of dapagliflozin | Example 11 | Comparative Example 5 |
|---|---|---|---|
| Purification method | / | Forming a cocrystal with sarcosine | Forming a solvate with (S)-1,2-propylene glycol/water |
| Batch number | 20200703-3 | 20200714-3 | 20200714-6 |
| Property | Yellow foamy solid | White solid | White solid |
| Yield (%) | / | 70 | 68 |
| HPLC normalized content (%) | 81.21 | 99.85 | 95.58 |
| Total content of impurities (%) | 18.79 | 0.15 | 4.42 |
| Number of impurities | 27 | 1 | 8 |
| Detected impurities | / | One impurity of 0.15% | 5 impurities exceeding 0.1%, maximal single impurity: 2.37% |
| Impurity removal effect | / | 26 impurities were completely removed; the removal rate of ring-opening impurities and dimer impurities is 100%, and the removal rate of five-membered ring impurities and isomer impurities is 96%. | 19 impurities were completely removed; the removal rate of ring-opening impurities is less than 20%, and the removal rate of five-membered ring impurities and isomer impurities is 83%. |

### Comparative Example 5:

600 ml of methyl tert-butyl ether was added to a 3000 mL three-neck flask, and stirring was started. 30 g of a crude product of dapagliflozin (batch number 20200703-3, yellow foamy solid, the detected impurities before purification are shown in table 8) prepared according to the method provided in patent document WO03099836A1 was added thereto and stirred until the solution was clarified. According to the method for preparing dapagliflozin (S)-propylene glycol monohydrate in Example 6 of patent document WO2008002824A1, 5.58 g of (S)-1,2-propylene glycol, 1.54 g of purified water, and 0.30 g of a seed crystal of dapagliflozin (S)-propylene glycol monohydrate were added thereto. The system was kept at 20°C to 30°C under stirring for 8 hours of crystallization, added with 1030 mL of cyclohexane, and then filtered. The collected solid was dried at 20°C to 25°C under vacuum for 4 hours to obtain 20 g of dapagliflozin (S)-propylene glycol monohydrate as an off-white solid, batch number: 20200714-6. The detected impurities after purification are shown in table 8.

### Comparative Example 6:

50 mL of absolute ethanol was added to a 100 mL three-neck flask, and stirring was started. 10 g of dapagliflozin (0.025 mol, HPLC: >99%) was added thereto and stirred until the solution was clarified. 2 mL of purified water was added to a 10 mL single-neck flask, and stirring was started. 2.20 g of L-alanine (0.025 mol) was added thereto and stirred until the solution was clarified. Then the resulting solution was added to the above solution of dapagliflozin in ethanol. The system was kept at -10°C to 10°C under stirring for 15 hours, with no solid precipitated. The solvent of the solution was removed under reduced pressure to obtain a white solid. The melting point was measured to be about 70°C with slightly unmelted particles, which was confirmed to be a physically mixed mixture of dapagliflozin and L-alanine instead of a cocrystal.

### Example 12: Preparation of canagliflozin-sarcosine cocrystal

25 mL of absolute ethanol was added to a 100 mL three-neck flask, and stirring was started. 5.00 g of canagliflozin (HPLC: >99%) was added thereto and stirred until the solution was clarified. 3 mL of purified water was added to a 10 mL single-neck flask, and stirring was started. 1.00 g of sarcosine was added thereto and stirred until the solution was clarified. Then the resulting solution was added to the above solution of canagliflozin in ethanol. The system was kept at 20°C to 30°C under stirring for 5 hours of crystallization, and then filtered. The collected solid was dried at 30°C to 40°C under vacuum for 6 hours to obtain 5.16 g of white solid (HPLC: 99.96%).

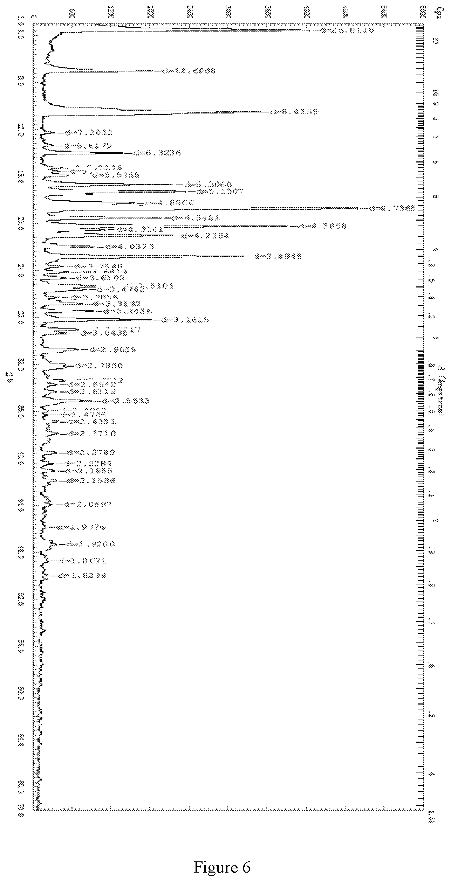

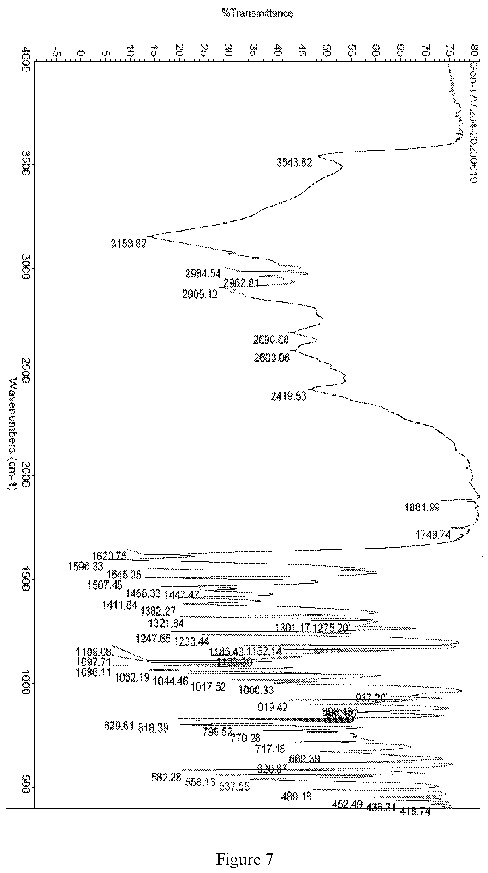

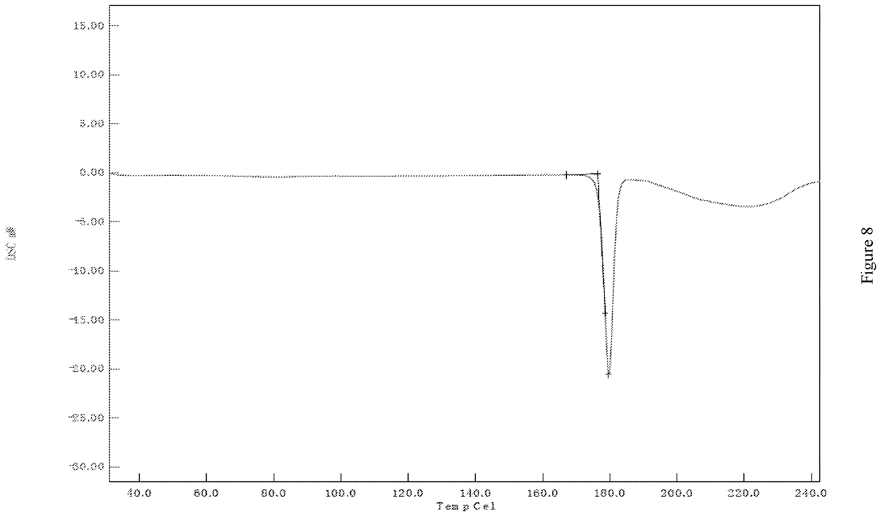

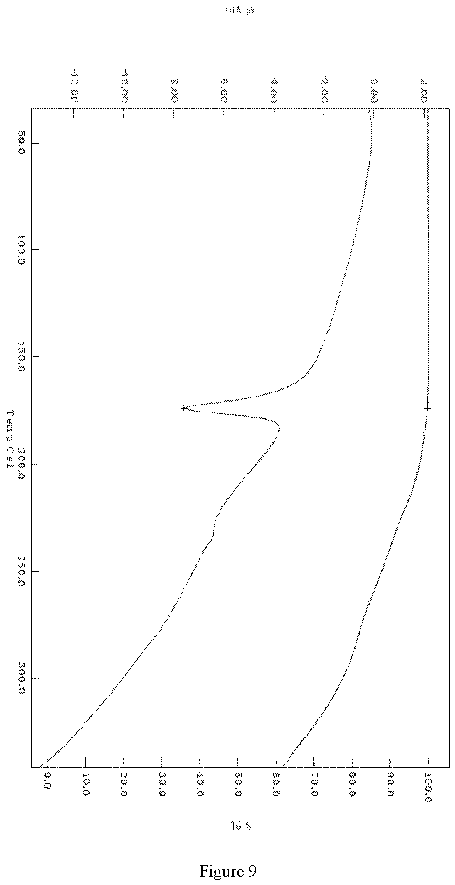

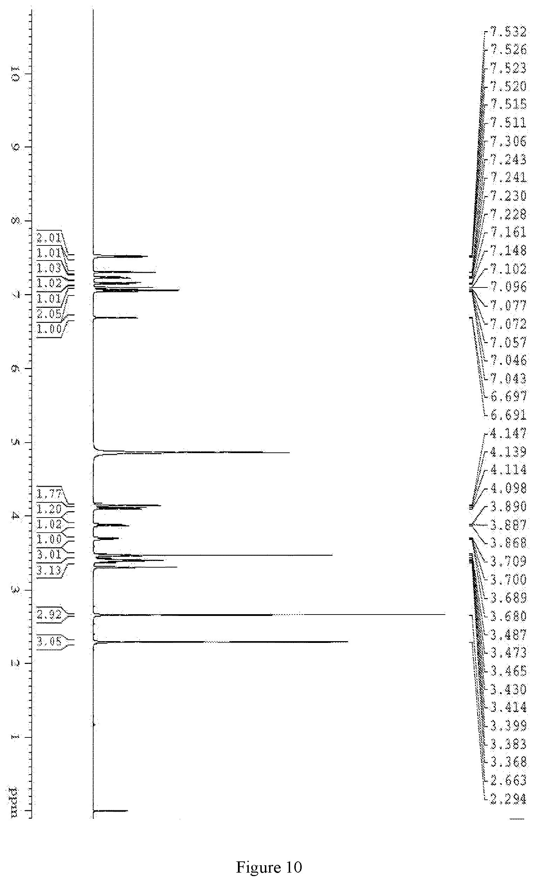

The white solid was confirmed as a canagliflozin-sarcosine cocrystal through X-ray powder diffraction (XRPD), single crystal diffraction, infrared spectroscopy (IR), differential scanning calorimetry (DSC), thermogravimetric analysis (TGA), and hydrogen nuclear magnetic resonance (¹H-NMR), see Figure 6 to Figure 10 for details..

¹HNMR (600MHz, MeOD) δ 7.535-7511 (m, 2H), 7.306 (s, 1H) 7.241-7.228 (d, 1H, J=7.8Hz), 7.161-7.148 (d, 1H, J=7.8Hz), 7.102-7.096 (d, 1H, J=3.6Hz) , 7.072-7043 (t, 2H, J=9.0Hz) , 6.697-6.691 (d, )H, J=3.6Hz), 4.174-4.098 (m, 3H), 3.887-3.868(d, 1H, J=11.4Hz), 3.709-3.680 (dd, 1H, J=12.0, 5.4Hz) , 3.487-3.465 (m, 3H) , 3.429-3.368 (m, 3H) , 2.663 (s, 3H) , 2.294 (s, 3H).

It can be seen that the molar ratio of canagliflozin to sarcosine is 1:1 according to the ¹HNMR spectrum data.

The canagliflozin-sarcosine cocrystal exhibits an X-ray powder diffraction (XRPD) spectrum represented by diffraction angle 20 using Cu-Kα radiation having characteristic peaks and the respective intensities as shown in the following table 9, where the diffraction angle 20 has an error of 0.2°:

**Table 9. Characteristic peaks and respective intensities of canagliflozin-sarcosine cocrystal in XRPD spectrum**

| No. | Diffraction angle 2θ | D | I/Io | No. | Diffraction angle 2θ | D | I/Io |
|---|---|---|---|---|---|---|---|
| 1 | 3.625 | 24.3531 | 67.7 | 11 | 20.296 | 4.3719 | 80.1 |
| 2 | 7.096 | 12.4468 | 26.4 | 12 | 20.597 | 4.3086 | 24.2 |
| 3 | 10.595 | 8.3430 | 62.8 | 13 | 21.110 | 4.2051 | 47.2 |
| 4 | 14.077 | 6.2861 | 22.7 | 14 | 22.062 | 4.0257 | 20.2 |
| 5 | 15.933 | 5.5579 | 130 | 15 | 22.887 | 3.8824 | 77.0 |
| 6 | 16.768 | 5.2829 | 47.7 | 16 | 25.414 | 3.5018 | 29.1 |
| 7 | 17.338 | 5.1104 | 42.7 | 17 | 27.544 | 3.2356 | 18.5 |
| 8 | 18.322 | 4.8381 | 31.2 | 18 | 28.287 | 3.1523 | 45.7 |
| 9 | 18.791 | 4.7184 | 100 | 19 | 33.763 | 2.6525 | 5.1 |
| 10 | 19.603 | 4.5248 | 48.2 | -- | -- | --- | - |

The canagliflozin-sarcosine cocrystal exhibits an infrared (IR) spectrum measured by the KBr pellet method having absorption peaks at 3543.82±10 cm⁻¹, 3153.82±10 cm⁻¹, 2690.68±5 cm⁻¹, 2603.06±5 cm⁻¹, 2419.53±5 cm⁻¹, 1596.33±5 cm⁻¹, 1507.48±5 cm⁻¹, 1411.84±5 cm⁻¹, 1382.27±5 cm⁻¹, 1321.84±5 cm⁻¹, 1233.44±5 cm⁻¹, 1086.11±5 cm⁻¹, 1062.19±5 cm⁻¹, 829.61±5 cm⁻¹, 799.52±5 cm⁻¹, and 537.55±5 cm⁻¹.

The canagliflozin-sarcosine cocrystal exhibits a differential scanning calorimetry (DSC) spectrum having an endothermic peak in the range of 160.0°C to 180.0°C with a peak value of 179.5°C as melting temperature.

The canagliflozin-sarcosine cocrystal exhibits a thermogravimetric analysis (TGA) spectrum having no measurable weight loss below 150°C and a TGA-DTA spectrum having a broad endothermic peak at 190°C to 230°C.

### Example 13

A single crystal of the dapagliflozin-sarcosine cocrystal was obtained by culturing in an ethanol/water mixed solvent system by a method of slow evaporation of solvent, and then characterized by single crystal X-ray diffraction.

The single crystal structure of the dapagliflozin-sarcosine cocrystal is shown in Figure 11.

**Table 10 Structure data and refinement parameters of structures of single crystal**

| | |
|---|---|
| Empirical formula | C₂₄H₃₂ClNO₈ |
| Formula weight | 497.95 |
| Temperature / K | 112.20(10) |
| Crystal system | orthorhombic |
| Space group | P2₁2₁2₁ |
| a/Å, b/Å, c/Å | 5.8339(7), 8.7909(10), 47.180(7) |
| α/°, β/°, γ/° | 90, 90, 90 |
| Volume / Å3 | 2419.6(5) |
| z | 4 |
| ρcalc / mg mm-3 | 1.367 |
| µ / mm-1 | 1.822 |
| F(000) | 1056 |
| Crystal size / mm3 | 0.550 × 0.470 × 0.190 |
| 2Θ range for data collection | 7.496 to 142.062° |
| Index ranges | -3 ≤ h ≤ 6, -10 ≤ k ≤ 10, -56 ≤ 1 ≤ 57 |
| Reflections collected | 7604 |
| Independent reflections | 4500[R(int) = 0.0555 (inf-0.9Å)] |
| Data/restraints/parameters | 4500/0/313 |
| Goodness-of-fit on F2 | 1.121 |
| Final R indexes [I>2σ (I) i.e. Fo>4σ (Fo)] | R1 = 0.0615, wR2 = 0.1768 |
| Final R indexes [all data] | R1 = 0.0749, wR2 = 0.1831 |
| Largest diff. peak/hole / e Å-3 | 0.412/-0.477 |
| Flack Parameters | 0.03(3) |
| Completeness | 0.9985 |

**Table 11 Classified atomic coordinate**

| Atom | **X** | Y | **Z** |
|---|---|---|---|
| C11 | -7182(3) | -8107.4(19) | -647.6(3) |
| 01 | -8732(8) | -155(5) | -162.7(9) |
| 08 | -3180(7) | 101(6) | -1868.7(9) |
| 03 | -1237(7) | -3339(5) | -1524.7(9) |
| 02 | -4307(8) | -6295(5) | -1930.6(8) |
| 07 | -4744(8) | -1431(5) | -1541.5(10) |
| 04 | 1151(8) | -3283(9) | -2038.1(10) |
| 06 | -2966(10) | -8370(7) | -2359.5(10) |
| C16 | -7078(12) | -2509(8) | -346.7(13) |
| C17 | -8771(11) | -1394(7) | -337.2(12) |
| C22 | -4852(11) | -408(7) | -1724.2(13) |
| C7 | -4751(11) | -5779(7) | -1444.3(12) |
| N1 | -9023(9) | -493(7) | -1629.0(11) |
| C8 | -3640(11) | -7062(7) | -1341.1(12) |
| C21 | -7086(13) | 1522(7) | 174.5(13) |
| C15 | -7308(11) | -3743(7) | -534.5(13) |
| C20 | -6743(12) | 55(8) | 14.4(13) |
| C6 | -4063(11) | -5096(7) | -1725.4(12) |
| C5 | -1605(10) | -4478(7) | -1734.1(12) |
| C11 | -7407(11) | -5855(7) | -1044.3(11) |
| C2 | -3907(11) | -5779(8) | -2214.4(12) |
| C18 | -10675(11) | -1530(8) | -514.6(13) |
| 05 | -1153(9) | -4729(11) | -2528.7(10) |
| C14 | -9196(11) | -3877(8) | -711.7(12) |
| C23 | -7161(11) | 287(7) | -1788.6(13) |
| C9 | -4365(11) | -7751(7) | -1091.8(12) |
| C10 | -6252(11) | -7149(8) | -949.1(12) |
| C13 | -9564(11) | -5221(7) | -907.3(12) |
| C4 | -1128(12) | -3904(10) | -2033.6(13) |
| C19 | -10885(11) | -2734(8) | -699.1(12) |
| C12 | -6588(10) | -5163(7) | -1294.8(12) |
| C3 | -1454(11) | -5194(11) | -2240.6(13) |
| C24 | -9140(11) | -136(9) | -1319.1(14) |
| C1 | -4458(13) | -7105(9) | -2405.5(13) |
| H3 | -2397 | -2776 | -1514 |
| H4 | 1366 | -2823 | -2192 |
| H6 | -3222 | -8744 | -2199 |
| H16 | -5773 | -2436 | -227 |
| H1C | -8841 | -1515 | -1649 |
| H1D | -10389 | -240 | -1709 |
| H8 | -2372 | -7473 | -1442 |
| H21A | -7245 | 2364 | 40 |
| H21B | -5760 | 1707 | 297 |
| H21C | -8475 | 1450 | 290 |
| H15 | -6147 | -4499 | -540 |
| H20A | -5340 | 112 | -103 |
| H20B | -6584 | -806 | 148 |
| H6A | -5150 | -4256 | -1774 |
| H5 | -535 | -5340 | -1695 |
| H2 | -4989 | -4927 | -2257 |
| H18 | -11845 | -780 | -508 |
| H5A | 161 | -4354 | -2549 |
| H23A | -7139 | 1378 | -1737 |
| H23B | -7469 | 209 | -1994 |
| H9 | -3588 | -8618 | -1020 |
| H13A | -10638 | -4912 | -1059 |
| H13B | -10309 | -6046 | -798 |
| H4A | -2244 | -3077 | -2080 |
| H19 | -12186 | -2796 | -820 |
| H12 | -7302 | -4262 | -1362 |
| H3A | -355 | -6032 | -2195 |
| H24A | -9248 | 968 | -1293 |
| H24B | -7756 | -516 | -1225 |
| H24C | -10493 | -626 | -1236 |
| H1A | -6061 | -7429 | -2372 |
| H1B | -4333 | -6775 | -2606 |

### Analysis result

1) Single crystal X-ray diffraction characterization and structural analysis indicate that the crystal belongs to the orthorhombic crystal system with space group of P212121, and its unit cell parameters are as follows:

**Table 12 Unit cell parameters**

| a (Å) | b (Å) | C (Å) | α° | β° | γ° |
|---|---|---|---|---|---|
| 5.8339(7) | 8.7909(10) | 47.180(7) | 90 | 90 | 90 |

2) The parameter Flack parameter for characterizing the absolute configuration is 0.03, which is less than 0.2. The absolute configuration of the chiral center of the dapagliflozin-sarcosine cocrystal determined through single crystal structure analysis is as follows: 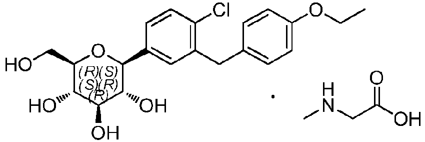
3) From the ellipsoid diagram of the molecular, it can be determined that it is composed of dapagliflozin and sarcosine at a ratio of 1: 1.
4) The following groups were bonded by hydrogen bonds to form cocrystal.

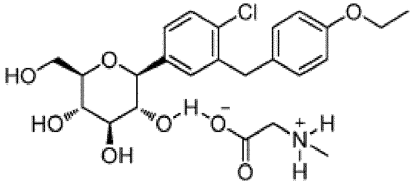

Test Example 1: Comparative experiment of influencing factors for dapagliflozin-sarcosine cocrystal and dapagliflozin (S)-propylene glycol monohydrate

The dapagliflozin-sarcosine cocrystal and dapagliflozin (S)-propylene glycol monohydrate were tested for influencing factors. The materials of the two crystal forms were directly exposed for 10 days to conditions of high temperature of 60°C±2°C, light irradiation of 4500LX±500LX, and high humidity RH75%±5%, to investigate the physical stability and chemical stability of the two crystal forms. The results are shown in Table 13, Table 14, and Table 15 below:

**Table 13 Test results of influencing factors for dapagliflozin-sarcosine cocrystal**

| Category | RT (min) | RRT | 0 day | Dapagliflozin-sarcosine cocrystal | | |
|---|---|---|---|---|---|---|
| | | | | 60°C | Light irradiation of 5000Lx | RH75% |
| Impurity 1 | 7.45 | 0.36 | / | / | / | / |
| Impurity 2 | 10.46 | 0.51 | / | / | / | / |
| Impurity 3 | 14.06 | 0.69 | / | / | / | / |
| Impurity 4 | 14.62 | 0.71 | / | / | / | / |
| Impurity5 | 16.02 | 0.78 | / | / | / | / |
| Impurity 6 | 21.65 | 1.06 | / | / | / | / |
| Impurity 7 | 22.01 | 1.07 | / | / | / | / |
| Impurity 8 | 27.17 | 1.33 | / | / | / | / |
| Impurity 9 | 30.53 | 1.49 | / | / | / | / |
| Impurity 10 | 33.01 | 1.61 | 0.03 | 0.02 | 0.02 | 0.02 |
| Impurity 11 | 33.72 | 1.65 | 0.01 | 0.01 | 0.01 | 0.01 |
| Impurity 12 | 34.69 | 1.69 | 0.03 | 0.02 | 0.02 | 0.02 |
| Impurity 13 | 39.10 | 1.91 | 0.01 | 0.01 | 0.01 | 0.01 |
| Total content of impurities (%) | | | 0.07 | 0.06 | 0.05 | 0.06 |
| Number of impurities | | | 4 | 4 | 4 | 4 |
| Property | | | White or off-white powder | White or off-white powder | White or off-white powder | White or off-white powder |

**Table 14 Test results of influencing factors for dapagliflozin (S)-propylene glycol**

| Category | RT (min) | RRT | 0 day | Dapagliflozin (S)-propylene glycol monohydrate | | |
|---|---|---|---|---|---|---|
| | | | | 60°C | Light irradiation of 5000Lx | RH75% |
| Impurity 1 | 7.45 | 0.36 | / | 0.02 | / | / |
| Impurity 2 | 10.46 | 0.51 | / | 0.14 | / | / |
| Impurity 3 | 14.06 | 0.69 | / | 0.01 | / | / |
| Impurity 4 | 14.62 | 0.71 | / | 0.21 | / | / |
| Impurity 5 | 16.02 | 0.78 | / | 0.01 | / | / |
| Impurity 6 | 21.65 | 1.06 | 0.01 | 0.01 | 0.01 | 0.01 |
| Impurity 7 | 22.01 | 1.07 | 0.01 | 0.01 | 0.01 | 0.01 |
| Impurity 8 | 27.17 | 1.33 | / | 0.02 | / | / |
| Impurity 9 | 30.53 | 1.49 | / | 0.02 | / | / |
| Impurity 10 | 33.01 | 1.61 | 0.02 | 0.02 | 0.02 | 0.02 |
| Impurity 11 | 33.72 | 1.65 | 0.01 | 0.01 | 0.01 | 0.01 |
| Impurity 12 | 34.69 | 1.69 | 0.01 | 0.01 | 0.01 | 0.01 |
| Impurity 13 | 39.10 | 1.91 | 0.02 | 0.02 | 0.02 | 0.02 |
| Total content of impurities (%) | | | 0.07 | 0.50 | 0.08 | 0.08 |
| Number of impurities | | | 6 | 13 | 6 | 6 |
| Property | | | White or off-white powder | Melted | White or off-white powder | White or off-white powder |

**Table 15 Crystal form stability of dapagliflozin-sarcosine cocrystal under influencing factors after 10 days**

| Diffraction angle 2θ of the characteristic diffraction peak (±0.2 °) | | | |
|---|---|---|---|
| 0 day | High temperature 60°C-10 days | Light radiation 5000LX- 10days | High humidity RH75%-10 days |
| 3.80 | 3.75 | 3.71 | 3.79 |
| 10.67 | 10.69 | 10.63 | 10.72 |
| 11.23 | 11.23 | 11.17 | 11.24 |
| 13.68 | 13.70 | 13.63 | 13.73 |
| 14.96 | 14.97 | 14.91 | 14.99 |
| 16.95 | 16.99 | 16.92 | 17.01 |
| 17.98 | 18.02 | 17.95 | 18.05 |
| 18.59 | 18.64 | 18.57 | 18.66 |
| 19.60 | 19.63 | 19.56 | 19.65 |
| 20.09 | 20.17 | 20.08 | 20.13 |
| 21.40 | 21.45 | 21.39 | 21.47 |
| 22.14 | 22.14 | 22.06 | 22.16 |
| 22.96 | 23.00 | 22.93 | 23.03 |
| 24.85 | 24.92 | 24.83 | 24.95 |
| 25.45 | 25.52 | 25.46 | 25.54 |
| 26.22 | 26.27 | 26.19 | 26.29 |
| 27.58 | 27.65 | 27.57 | 27.67 |
| 33.57 | 33.65 | 33.61 | 33.69 |

According to the above test results of influencing factors, it can be seen that for dapagliflozin-sarcosine cocrystal, the number of impurities detected and the total content of impurities detected under all the test conditions did not increase. In contrast, for dapagliflozin (S)-propylene glycol monohydrate, both of the number of impurities detected and the total content of impurities detected at high temperature of 60°C increased, with the number of impurities detected rising from 6 at 0 day to 13. However, the number of impurities and the content of impurities detected for dapagliflozin-sarcosine cocrystal under each influencing factor after 10 days were consistent with those at 0 day, showing no trend of change. According to the XRPD results of samples under each influencing factor at 0 day and after 10 days, the crystal form of dapagliflozin-sarcosine cocrystal tested under each influencing factor for 10 days did not change.

Test Example 2: Comparative experiment of influencing factors for canagliflozin-sarcosine cocrystal and canagliflozin hemihydrate

The canagliflozin-sarcosine cocrystal and canagliflozin hemihydrate (original crystal form) were tested for influencing factors. The materials of the two crystal forms were directly exposed for 10 days to conditions of high temperature of 60°C±2°C, light irradiation of 4500LX±500LX, and high humidity RH75%±5%, to investigate the physical stability and chemical stability of the two crystal forms. The results are shown in Table 16 and Table 17 below:

**Table 16 Comparison results of influencing factors for canagliflozin-sarcosine cocrystal and canagliflozin hemihydrate**

| Category | 0 day | | Directly exposed at 60°C for 10 days | | RH75% for 10 days | | 5000LX for 10 days | |
|---|---|---|---|---|---|---|---|---|
| | cocrystal | hemihydrate | cocrystal | hemihydrate | cocrystal | hemihydrate | cocrystal | hemihydrate |
| RRT0.57 | 0.01 | 0.01 | 0.01 | 0.01 | 0.01 | 0.01 | 0.05 | 0.02 |
| RRT0.67 | / | 0.01 | / | 0.01 | / | 0.01 | / | / |
| RRT0.73 | / | / | / | / | / | / | 0.01 | 0.01 |
| RRT0.93 | 0.01 | 0.01 | 0.01 | 0.01 | 0.01 | 0.02 | 0.01 | 0.01 |
| RRT1.12 | / | / | / | / | / | / | 0.01 | 0.01 |
| RRT1.17 | / | 0.01 | / | 0.01 | / | 0.01 | / | 0.01 |
| RRT2.25 | 0.01 | 0.01 | 0.01 | 0.01 | 0.01 | 0.01 | 0.01 | 0.01 |
| RRT2.28 | / | 0.01 | / | 0.01 | / | 0.01 | / | 0.01 |
| Total content of impurities | 0.03 | 0.06 | 0.03 | 0.05 | 0.03 | 0.07 | 0.09 | 0.08 |
| Number of impurities | 3 | 6 | 3 | 6 | 3 | 6 | 5 | 7 |
| Property | White powder | White powder | White powder | White powder | White powder | White powder | White powder | White powder |

According to the above test results of influencing factors, it can be seen that for canagliflozin-sarcosine cocrystal, the number of impurities detected and the total content of impurities detected did not show an increasing trend under the conditions of high temperature 60°C and high humidity of RH75% for 10 days compared with the test results at 0 day. Under the condition of light irradiation of 5000LX for 10 days, 2 new impurities were detected in canagliflozin-sarcosine cocrystal, with a maximum detected amount of about 0.05%, and under the condition of light irradiation, 1 new impurity was detected in hemihydrate, indicating that the chemical stability of canagliflozin-sarcosine cocrystal is not worse than that of canagliflozin hemihydrate. The canagliflozin hemihydrate lost weight by about 1.0% under the condition of high temperature of 60°C for 10 days and gained weight by about 2.1% under the condition of high humidity of RH75% for 10 days. In contrast, the weight of the canagliflozin-sarcosine cocrystal basically remained unchanged under the conditions of high temperature of 60°C and high humidity of RH75%, indicating that the crystal form of canagliflozin-sarcosine cocrystal prepared by the present invention has a better physical stability than that of canagliflozin hemihydrate.

**Table 17 Crystal form stability of canagliflozin-sarcosine cocrystal under influencing factors after 10 days**

| Diffraction angle 2θ of the characteristic diffraction peak (±0.2 °) | | | |
|---|---|---|---|
| 0 day | High temperature 60°C-10 days | Light radiation 5000LX- 10 days | High humidity RH75%-10 days |
| 3.63 | 3.59 | 3.61 | 3.53 |
| 7.10 | 7.07 | 7.06 | 7.01 |
| 10.60 | 10.58 | 10.56 | 10.50 |
| 14.08 | 14.06 | 14.05 | 14.00 |
| 16.77 | 16.77 | 16.75 | 16.70 |
| 17.34 | 17.34 | 17.33 | 17.27 |
| 18.32 | 18.32 | 18.32 | 18.24 |
| 18.79 | 18.79 | 18.78 | 18.72 |
| 19.60 | 19.60 | 19.59 | 19.53 |
| 20.30 | 20.30 | 20.29 | 20.23 |
| 20.60 | 20.59 | 20.59 | 20.53 |
| 21.11 | 21.11 | 21.11 | 21.05 |
| 22.06 | 22.06 | 22.05 | 22.00 |
| 22.89 | 22.89 | 22.87 | 22.81 |
| 25.41 | 25.41 | 25.41 | 25.34 |
| 28.29 | 28.28 | 28.27 | 28.21 |
| 33.72 | 33.79 | 33.73 | 33.72 |

According to the XRPD results of samples under each influencing factor at 0 day and after 10 days, the characteristic diffraction angles of the canagliflozin cocrystal tested under each influencing factor for 10 days were basically the same as that of the 0 day. Therefore, the crystal form did not change.

### Test Example 3: Hygroscopicity test of dapagliflozin-sarcosine cocrystal

The dapagliflozin-sarcosine cocrystal was tested for hygroscopicity, and the results are shown in Table 18 below:

**Table 18 Test results of hygroscopicity of dapagliflozin-sarcosine cocrystal**

| Weighing bottle (g) | Weighing bottle+sample (g) | RH80%/25°C 24h | Percentage of weight gain (%) |
|---|---|---|---|
| | | Weighing bottle+sample (g) | |
| 33.4198 | 34.4046 | 34.4047 | 0.00 |

According to the above data, it can be seen that dapagliflozin-sarcosine cocrystal does not have hygroscopicity.

### Test Example 4: Comparison of sieving performance

Each 30 g of dapagliflozin-sarcosine cocrystal and dapagliflozin (S)-propylene glycol hydrate was sieved by a round sieve with a diameter of 20 cm and a mesh number of 80 using a 8411 type electric vibrating sieve (Daoxu Yuezhou Tugong Instrument Factory, Shangyu District, Shaoxing City) at a rotating speed of 1400 rpm for 20 minutes. The materials in the tray below the sieve was collected, and the situations after sieving including the residue of materials and large particle materials on the sieve, and the adsorption of materials by the sieve were observed. The results are shown in Table 19 below:

**Table 19 Comparison results of sieving**

| Sample | Yield (%, n=3) | Average yield (%) | Situations after sieving |
|---|---|---|---|
| Dapagliflozin-sarcosine cocrystal | 93.6, 90.5, 91.3 | 91.8 | Almost no large particles on the sieve and almost no adsorption on the sieve; almost no electrostatic force when collecting the fine powder in the tray. |
| Dapagliflozin (S)-propylene glycol hydrate | 85.2, 87.7, 84.9 | 86.0 | Almost no large particles but a small amount of material was adsorbed on the siev; the electrostatic force was strong and fluttering occurred when collecting the fine powder in the tray. |

The above yield results were subjected to one-way ANOVA, and the results are shown in Table 20 below:

**Table 20 ANOVA results of yield after sieving**

| Source of difference | SS | df | MS | F | P-value |
|---|---|---|---|---|---|
| Between-groups | 51.627 | 1 | 51.627 | 20.85 | 0.01 |

It can be seen from the above data of ANOVA results of yield after sieving, the two materials have a significant difference.

According to the yield after sieving and the observed situations after sieving, it can be seen that the powder state of dapagliflozin-sarcosine cocrystal is more conducive to sieving, with small sieving loss, high yield and basically no electrostatic effect.

Test Example 5: Comparison of particle size and fluidity of dapagliflozin-sarcosine cocrystal and dapagliflozin-(S)-propylene glycol monohydrate

The dapagliflozin-sarcosine cocrystal and dapagliflozin-(S)-propylene glycol monohydrate were detected by a laser particle size analyzer using water as a dispersant. The angle of repose, bulk density and tap density of the dapagliflozin-sarcosine cocrystal and dapagliflozin-(S)-propylene glycol monohydrate were measured by BT-1000 powder comprehensive property tester. The results are shown in Table 21 and Table 22 below:

**Table 21 Test results of particle size**

| Sample | D10 (µm) | D50 (µm) | D90 (µm) |
|---|---|---|---|
| Dapagliflozin-sarcosine cocrystal | 5.151 | 24.83 | 89.93 |
| Dapagliflozin-(S)-propylene glycol monohydrate | 2.492 | 61.55 | 256.2 |

According to the above results, it can be seen that the D90 of the dapagliflozin cocrystal is 89.93 µm. Such particle size distribution is conducive to the uniform mixing of pharmaceutical compositions in the preparation process, especially for small-scale preparations.

**Table 22 Test results for fluidity**

| Sample | Angle of repose (°) | Bulk density (g/ml) | Tap density (g/ml) |
|---|---|---|---|
| Dapagliflozin-sarcosine cocrystal | 40 | 0.40 | 0.58 |
| Dapagliflozin-(S)-propylene glycol monohydrate | 48 | 0.35 | 0.53 |

According to the above results, it can be seen that the bulk density and tap density of the dapagliflozin-sarcosine cocrystal and dapagliflozin-(S)-propylene glycol monohydrate were slightly different. The dapagliflozin-sarcosine cocrystal had a smaller angle of repose, and the fluidity was better than that of dapagliflozin-(S)-propylene glycol monohydrate.

Test Example 6: Comparison of difficulty of mixing in production process of preparation

The components shown in Table 20 were mixed by FH laboratory type mixer (0.5 L hopper) at a rotation speed of 8 rpm for 30 minutes. A total of 5 samples in different positions of the hopper were taken to detect the content of the main drug, and RSD was calculated to evaluate the difficulty of mixing. The results are shown in Table 23.

**Table 23 Components of formula**

| Components | Amount (g) |
|---|---|
| Main drug (calculated by dapagliflozin) | 10.0 |
| Microcrystalline cellulose | 171.5 |
| Anhydrous lactose | 50.0 |
| Cross-linked povidone | 10.0 |
| Cross-linked sodium carboxymethylcellulose | 4.0 |
| Silicon dioxide | 3.75 |
| Magnesium stearate | 2.5 |

**Table 24 Test results for fluidity**

| Sample | Dapagliflozin-sarcosine cocrystal | Dapagliflozin-(S)-propylene glycol monohydrate |
|---|---|---|
| RSD (%) | 1.22 | 8.89 |

According to the above results, it can be seen that with the same formula and the same mixing process, the formula with the dapagliflozin-sarcosine cocrystal as the main drug had the best mixing uniformity, which met the production requirements of preparations. The dapagliflozin-(S)-propylene glycol monohydrate tended to agglomerate and was difficult to mix evenly.

Test Example 7: Solubility of dapagliflozin-sarcosine cocrystal and canagliflozin-sarcosine cocrystal in different pH buffer salt solutions

Appropriate amount of the dapagliflozin-sarcosine cocrystal and canagliflozin-sarcosine cocrystal were added respectively to hydrochloric acid of pH 1.0, citrate buffer of pH 3.0, acetate buffer of pH 4.0, phosphate buffer of pH 6.8, water, artificial gastric juice, artificial intestinal juice, placed on a shaker with a constant temperature air bath at 25°C, and centrifuged at 13000 rpm/min for 10 minutes at 12h and 24h, respectively. The supernatant was used for analysis, and the results are shown in Table 25 below:

**Table 25 Solubility (25°C)**

| Medium | Solubility in different pH media | | | |
|---|---|---|---|---|
| | Dapagliflozin-sarcosine cocrystal (mg/ml) | | Canagliflozin-sarcosine cocrystal (mg/ml) | |
| | 12h | 24h | 12h | 24h |
| Hydrochloric acid of pH 1.0 | 1.60 | 1.56 | 34.92 | 35.12 |
| Citrate buffer of pH 3.0 | 1.62 | 1.61 | 33.87 | 34.23 |
| Acetate buffer of pH 4.0 | 1.64 | 1.62 | 32.65 | 32.66 |
| Phosphate buffer of pH 6.8 | 1.58 | 1.58 | 31.28 | 33.34 |
| Water | 1.60 | 1.59 | 35.12 | 34.56 |
| Artificial gastric juice | 1.56 | 1.55 | 31.16 | 32.08 |
| Artificial intestinal juice | 1.40 | 1.43 | 30.35 | 30.89 |

According to the measurement results, although the dapagliflozin-sarcosine cocrystal has a melting point higher than the dapagliflozin-(S)-propylene glycol monohydrate, its solubility in different pH media remains between 1.4 mg/ml to 1.6 mg/ml, which is consistent with the solubility of the dapagliflozin-(S)-propylene glycol monohydrate reported in the literature. The solubility of the canagliflozin-sarcosine cocrystal in different pH media is about 30 µg/ml, almost insoluble. According to the Japanese IF document about canagliflozin, the canagliflozin hemihydrate is almost insoluble in water (1 g cannot be dissolved in 10000 ml), and the solubility of the two in aqueous solution is basically the same. However, the canagliflozin cocrystal is more suitable for the industrialized large-scale production of preparation compositions due to its physical and chemical properties and the physical stability of the crystal form.

The descriptions of the above examples are only used to help understand the method and core idea of the present invention. It should be noted that for those skilled in the art, improvements and modifications can be made to the present invention without departing from the principle of the present invention, and these improvements and modifications also fall within the protection scope of the claims of the present invention.

## Claims

1. An SGLT-2 inhibitor-sarcosine cocrystal.

2. The SGLT-2 inhibitor-sarcosine cocrystal according to claim 1, wherein its X-ray powder diffraction pattern shows diffraction peaks at 2θ±0.2° of 10.6±0.2°, 19.6±0.2°, 22.1±0.2°, and 33.6±0.2°.

3. The SGLT-2 inhibitor-sarcosine cocrystal according to claim 1, wherein its infrared spectrum shows characteristic absorption peaks at least at 3540±10 cm⁻¹, 2691±5 cm⁻¹, 2603 ±5 cm⁻¹, and 2420±5 cm⁻¹.

4. The SGLT-2 inhibitor-sarcosine cocrystal according to claim 1, wherein its thermogravimetric analysis curve shows a broad endothermic peak at 190°C to 230°C.

5. The SGLT-2 inhibitor-sarcosine cocrystal according to claim 1, wherein its hydrogen nuclear magnetic resonance spectrum (HNMR, 600MHz, MeOD) shows a -CH₃ peak in a chemical shift range of 2.4 ppm to 3.2 ppm and a -CH₂- peak in a chemical shift range of 3.0 ppm to 4.0 ppm in addition to a resonance peak of the SGLT-2 inhibitor.

6. The SGLT-2 inhibitor-sarcosine cocrystal according to claim 1, wherein the SGLT-2 inhibitor is selected from the group consisting of dapagliflozin, empagliflozin, canagliflozin, tofogliflozin, ipragliflozin, luseogliflozin, sotagliflozin, janagliflozin, bexagliflozin, alligliflozin, ertugliflozin, henagliflozin, remogliflozin, tianagliflozin, wangeliejing, and a combination thereof, or the SGLT-2 inhibitor has a structure represented by Formula a:

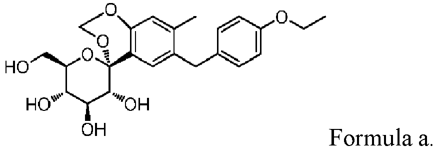

7. The SGLT-2 inhibitor-sarcosine cocrystal according to claim 1, wherein the SGLT-2 inhibitor-sarcosine cocrystal is free of a single impurity exceeding 0.1%.

8. The SGLT-2 inhibitor-sarcosine cocrystal according to claim 1, having a structure represented by Formula I: 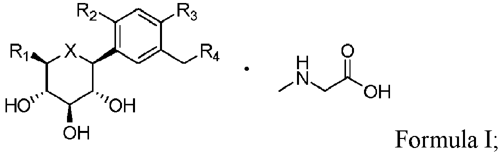 wherein R₁, R₂, R₃, R₄, and X are listed in the following table:
| Number | 1 | 2 | 3 | 4 | 5 |
|---|---|---|---|---|---|
| R₁ | 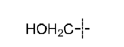 | 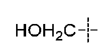 | 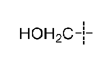 | 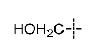 | 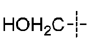 |
| R₂ | 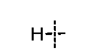 | 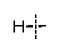 | 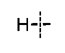 | 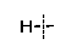 | 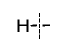 |
| R₃ | 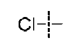 | 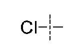 | 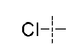 | 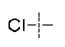 | 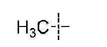 |
| R₄ | 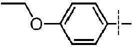 | 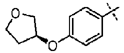 | 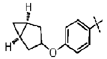 | 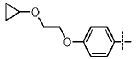 | 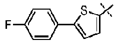 |
| X | O | O | O | O | O |
| Number | 6 | 7 | 8 | 9 | 10 |
|---|---|---|---|---|---|
| R₁ | 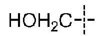 | 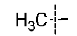 | 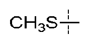 | 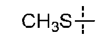 | 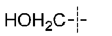 |
| R₂ | 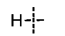 | 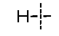 | 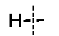 | 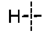 | 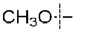 |
| R₃ | 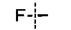 | 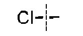 | 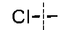 | 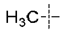 | 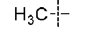 |
| R₄ | 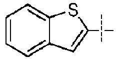 | 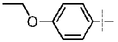 | 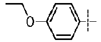 | 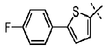 | 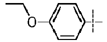 |
| X | O | O | O | O | S |

9. The SGLT-2 inhibitor-sarcosine cocrystal according to claim 1, having a structure represented by Formula II: 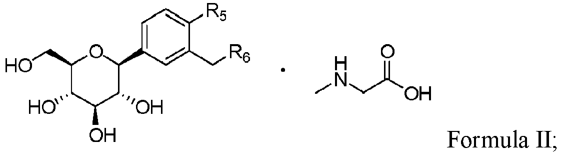 wherein R₅ is Cl, and R₆ is selected from the group consisting of: 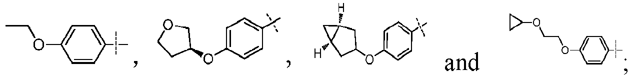 or R₅ is methyl, R₆ is 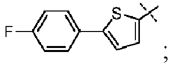 or R₅ is F, R₆ is

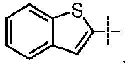

10. The SGLT-2 inhibitor-sarcosine cocrystal according to claim 1, wherein a molar ratio of the SGLT-2 inhibitor to sarcosine is 1:(0.5-5.0), and in some embodiments, a molar ratio of the SGLT-2 inhibitor to sarcosine is preferably 1:0.6, 1:0.7, 1:0.8, 1:0.9, 1:0.95, 1:1.0, 1:1.05, 1:1.1, 1:1.2, 1:1.3, 1:1.4, 1:1.5, 1:1.6, 1:1.7, 1:1.8, 1:1.9 or 1:2.0; more preferably, a molar ratio of the SGLT-2 inhibitor to sarcosine is 1:0.8, 1:0.9, 1:0.95, 1:1.0, 1:1.05, 1:1.1, 1:1.2, 1:1.3, 1:1.4 or 1:1.5.

11. The SGLT-2 inhibitor-sarcosine cocrystal according to claim 1, wherein the SGLT-2 inhibitor is dapagliflozin, and the SGLT-2 inhibitor-sarcosine cocrystal exhibits an X-ray powder diffraction pattern having diffraction peaks at 2θ±0.2° of 3.8±0.2°, 10.6±0.2°, 13.7±0.2°, 17.0±0.2°, 18.0±0.2°, 18.6±0.2°, 19.6±0.2°, 20.1±0.2°, 21.4±0.2°, 22.1±0.2°, 23.0±0.2°, 25.4±0.2°, 27.6±0.2° and 33.6±0.2°.

12. The SGLT-2 inhibitor-sarcosine cocrystal according to claim 11, wherein a single crystal of the dapagliflozin sarcosine cocrystal obtained by culturing in an ethanol/water mixed solvent system by slow evaporation of solvent has unit cell parameters of:
unit cell size:
a=5.8339(7) Å, b=8.7909(10) Å, c=47.180(7) Å;
α=90°, β=90°, γ=90°;
space group=P212121;
molecule/asymmetric unit=4;
wherein the structure of the single crystal of the cocrystal is measured at T=112 with classified atomic coordinates listed in Table 11.

13. The SGLT-2 inhibitor-sarcosine cocrystal according to claim 11, wherein its infrared spectrum shows characteristic absorption peaks at 3543.67±10 cm⁻¹, 3161.94±10 cm⁻¹, 2690.95±5 cm⁻¹, 2603.78±5 cm⁻¹, 2419.39±5 cm⁻¹, 2360.665 cm⁻¹, 1599.61±5 cm⁻¹, 1510.92±5 cm⁻¹, 1291.19±5 cm⁻¹, and 1045.97 cm⁻¹.

14. The SGLT-2 inhibitor-sarcosine cocrystal according to claim 11, wherein its DSC spectrum shows an endothermic peak in a range of 140.0°C to 155.0°C with a peak value of 149.0°C as melting temperature.

15. The SGLT-2 inhibitor-sarcosine cocrystal according to claim 11, wherein its TGA-DTA spectrum shows no measurable weight loss below 150°C, but shows a broad endothermic peak in a range of 190°C to 230°C.

16. The SGLT-2 inhibitor-sarcosine cocrystal according to claim 11, wherein its hydrogen nuclear magnetic resonance spectrum (HNMR, 600MHz, MeOD) shows resonance peaks at δ 7.343-7.329 (d, 1H, J=8.4Hz), 7.315-7.312 (d, 1H, J=1.8Hz), 7.276-7.259 (dd, 1H, J=8.4, 1.8Hz), 7.091-7.077 (d, 2H, J=8.4Hz), 6.795-6.781 (d, 2H, J=8.4Hz), 4.089-4.073 (d, 1H, J=9.6Hz), 4.055-3.977 (q, 2H, J=15.0Hz), 3.994-3.959 (q, 2H, J=7.2Hz), 3.876-3.854 (dd, 1H, J=12.0, 1.8Hz), 3.697-3.668 (dd, 1H, J=12.0, 5.4Hz), 3.467 (s, 2H) 3.461-3.431 (m, 1H), 3.409-3.370 (m, 2H), 3.283-3.268 (m, 1H), 2.665 (s, 3H), and 1.360-1.337 (t, 3H, J=7.2Hz).

17. The SGLT-2 inhibitor-sarcosine cocrystal according to claim 1, wherein the SGLT-2 inhibitor is canagliflozin, and the SGLT-2 inhibitor-sarcosine cocrystal exhibits an X-ray powder diffraction pattern having diffraction peaks at 2θ±0.2° of 3.6±0.2°, 7.1±0.2°, 10.6±0.2°, 14.1±0.2°, 16.8±0.2°, 17.3±0.2°, 18.3±0.2°, 18.8±0.2°, 19.6±0.2°, 20.3±0.2°, 21.1±0.2°, 22.1±0.2°, 22.9±0.2°, 25.4±0.2°, 28.2±0.2°, and 33.6±0.2°.

18. The SGLT-2 inhibitor-sarcosine cocrystal according to claim 17, wherein its infrared spectrum shows characteristic absorption peaks at 3543.82±10 cm⁻¹, 3153.82±10 cm⁻¹, 2690.68±5 cm⁻¹, 2603.06±5 cm⁻¹, 2419.53±5 cm⁻¹, 1596.33±5 cm⁻¹, 1507.48±5 cm⁻¹, 1086.11±5 cm⁻¹, 1062.19±5 cm⁻¹, and 829.61±5 cm⁻¹.

19. The SGLT-2 inhibitor-sarcosine cocrystal according to claim 17, wherein its DSC spectrum shows an endothermic peak in a range of 160.0°C to 180.0°C with a peak value of 179.5°C as melting temperature.

20. The SGLT-2 inhibitor-sarcosine cocrystal according to claim 17, wherein its TGA-DTA spectrum shows no measurable weight loss below 150°C, but shows a broad endothermic peaks in a range of 190°C to 230°C.

21. The SGLT-2 inhibitor-sarcosine cocrystal according to claim 1, wherein its hydrogen nuclear magnetic resonance spectrum (HNMR, 600MHz, MeOD) shows resonance peaks at δ 7.535-7.511 (m, 2H), 7.306 (s, 1H), 7.241-7.228 (d, 1H, J=7.8Hz), 7.161-7.148 (d, 1H, J=7.8Hz), 7.102-7.096 (d, 1H, J=3.6Hz), 7.072-7.043 (t, 2H, J=9.0Hz), 6.697-6.691 (d, 1H, J=3.6Hz), 4.174-4.098 (m, 3H), 3.887-3.868 (d, 1H, J=11.4Hz), 3.709-3.680 (dd, 1H, J=12.0, 5.4Hz), 3.487-3.465 (m, 3H), 3.429-3.368 (m, 3H), 2.663 (s, 3H), and 2.294 (s, 3H).

22. A method for preparing an SGLT-2 inhibitor-sarcosine cocrystal, comprising:
mixing a solution of an SGLT-2 inhibitor with a solution of sarcosine, performing static crystallization or cooling crystallization, and performing solid-liquid separation to obtain the SGLT-2 inhibitor-sarcosine cocrystal.

23. The method according to claim 22, wherein a molar ratio of the SGLT-2 inhibitor to sarcosine is 1:(0.5-5.0), and in some embodiments, a molar ratio of the SGLT-2 inhibitor to sarcosine is preferably 1:0.6, 1:0.7, 1:0.8, 1:0.9, 1:0.95, 1:1.0, 1:1.05, 1:1.1, 1:1.2, 1:1.3, 1:1.4, 1:1.5, 1:1.6, 1:1.7, 1:1.8, 1:1.9 or 1:2.0.

24. The method according to claim 22, wherein a solvent in the solution of the SGLT-2 inhibitor is selected from the group consisting of C1-C10 alcohol, C3-C10 ketone, ether, nitrile, and a combination thereof; and
a solvent in the solution of sarcosine is water.

25. The method according to claim 22, wherein the static crystallization or the cooling crystallization is carried out at a temperature of -20°C to 40°C; and in some embodiments, the crystallization is preferably carried out at a temperature of -15°C to 35°C, -10°C to 30°C, -5°C to 30°C, 0°C to 30°C, 5°C to 30°C, 10°C to 30°C, 15°C to 30°C, or 20°C to 30°C.

26. The method according to claim 22, wherein the static crystallization or the cooling crystallization is carried out for 4 hours to 48 hours, preferably 4 hours to 24 hours, 4 hours to 16 hours, 4 hours to 12 hours, and more preferably 8 hours to 12 hours.

27. A method for purifying a crude product of an SGLT-2 inhibitor, comprising:
mixing the crude product of the SGLT-2 inhibitor with sarcosine, stirring at room temperature, and performing solid-liquid separation to obtain a complex of the SGLT-2 inhibitor and sarcosine; and
dissociating the complex of the SGLT-2 inhibitor and sarcosine to obtain a pure product of the SGLT-2 inhibitor in free state.

28. The method according to claim 27, wherein a molar ratio of the crude product of the SGLT-2 inhibitor to sarcosine is 1:(0.5-5.0), and in some embodiments, a molar ratio of the crude product of the SGLT-2 inhibitor to sarcosine is preferably 1:0.7, 1:0.8, 1:0.9, 1:0.95, 1:1.0, 1:1.05, 1:1.1, 1:1.2, 1:1.3, 1:1.4, 1:1.5, 1:1.6, 1:1.7, 1:1.8, 1:1.9, 1:2.0 or 1:3.0; more preferably, a molar ratio of the crude product of the SGLT-2 inhibitor to sarcosine is 1:1.0, 1:1.1, 1:1.2, 1:1.3, 1:1.4, 1:1.5, 1:1.6, 1:1.7, 1:1.8, 1:1.9, 1:2.0 or 1:3.0.

29. The method according to claim 27, further comprising:
using the pure product of the SGLT-2 inhibitor in the free state as a raw material to directly prepare a final pharmaceutically acceptable crystal form of the SGLT-2 inhibitor; or
performing recrystallization or co-crystallization by using the pure product of the SGLT-2 inhibitor in the free state as a raw material to prepare a final pharmaceutically acceptable crystal form of the SGLT-2 inhibitor.

30. The method according to claim 29, wherein the final pharmaceutically acceptable crystal form of the SGLT-2 inhibitor is selected from the group consisting of a pure product, solvate, hydrate, hydrate of solvate, cocrystal and double salt of the SGLT-2 inhibitor.

31. The method according to claim 27, wherein the pure product of the SGLT-2 inhibitor in the free state has a HPLC normalized purity no less than 99%.

32. A pharmaceutical composition, comprising:
the SGLT-2 inhibitor-sarcosine cocrystal according to any one of claims 1 to 21, the SGLT-2 inhibitor-sarcosine cocrystal prepared by the method according to any one of claims 22 to 26, or the pure product of the SGLT-2 inhibitor in the free state obtained by the method according to any one of claims 27 to 31, and
a pharmaceutically acceptable carrier, excipient, diluent, adjuvant, vehicle or a combination thereof.

33. Use of the SGLT-2 inhibitor-sarcosine cocrystal according to any one of claims 1 to 21, the SGLT-2 inhibitor-sarcosine cocrystal prepared by the method according to any one of claims 22 to 26, the pure product of the SGLT-2 inhibitor in the free state obtained by the method according to any one of claims 27 to 31, or the pharmaceutical composition according to claim 32 in the manufacture of a medicament for preventing, treating or alleviating a cardiovascular and cerebrovascular disease, diabetes or a complication thereof, or nephropathy caused by a non-diabetic disease.

34. The use according to claim 33, wherein the diabetes and the complication thereof is selected from the group consisting of essential hypertension, type 2 diabetes with hypertension, nephropathy with type 2 diabetes, nephropathy with hypertension and diabetes, nephropathy, type 1 diabetes, nephropathy with type 1 diabetic, liver fibrosis, insulin resistance, hyperglycemia, hyperinsulinemia, elevated fatty acid or glycerol level in blood, hyperlipidemia, dyslipidemia, obesity, and a combination thereof.

35. Use of the SGLT-2 inhibitor-sarcosine cocrystal according to any one of claims 1 to 21, the SGLT-2 inhibitor-sarcosine cocrystal prepared by the method according to any one of claims 22 to 26, the pure product of the SGLT-2 inhibitor in the free state obtained by the method according to any one of claims 27 to 31, or the pharmaceutical composition according to claim 32 in the manufacture of a medicament for lowering blood pressure.

36. A method for preventing, treating or alleviating diabetes or a complication thereof, comprising:
contacting the SGLT-2 inhibitor-sarcosine cocrystal according to any one of claims 1 to 21, the SGLT-2 inhibitor-sarcosine cocrystal prepared by the method according to any one of claims 22 to 26, the pure product of the SGLT-2 inhibitor in the free state obtained by the method according to any one of claims 27 to 31, or the pharmaceutical composition according to claim 32 with a biological specimen.

37. A method for lowering blood pressure, comprising:
contacting the SGLT-2 inhibitor-sarcosine cocrystal according to any one of claims 1 to 21, the SGLT-2 inhibitor-sarcosine cocrystal prepared by the method according to any one of claims 22 to 26, the pure product of the SGLT-2 inhibitor in the free state obtained by the method according to any one of claims 27 to 31, or the pharmaceutical composition according to claim 32 with a biological specimen.

38. A method for treating nephropathy caused by a non-diabetic disease, comprising:
contacting the SGLT-2 inhibitor-sarcosine cocrystal according to any one of claims 1 to 21, the SGLT-2 inhibitor-sarcosine cocrystal prepared by the method according to any one of claims 22 to 26, the pure product of the SGLT-2 inhibitor in the free state obtained by the method according to any one of claims 27 to 31, or the pharmaceutical composition according to claim 32 with a biological specimen.
